(19) Europäisches Patentamt
European Patent Office
Office européen des brevets

(11) EP 1 923 397 B1

(12) # EUROPEAN PATENT SPECIFICATION

(45) Date of publication and mention of the grant of the patent:
**28.10.2009 Bulletin 2009/44**

(51) Int Cl.:
*C07K 7/00* (2006.01) *C07K 1/107* (2006.01)
*C12Q 1/00* (2006.01) *G01N 33/52* (2006.01)
*G01N 33/573* (2006.01) *C12Q 1/37* (2006.01)

(21) Application number: **07120340.0**

(22) Date of filing: **09.11.2007**

(54) **Fluorinated amino acids and peptides**

Fluorierte Aminosäuren und Peptide

Acides aminés et peptides fluorés

(84) Designated Contracting States:
**DE FR GB IT**

(30) Priority: **14.11.2006 EP 06124067**

(43) Date of publication of application:
**21.05.2008 Bulletin 2008/21**

(73) Proprietor: **NERVIANO MEDICAL SCIENCES S.r.l.**
**20014 Nerviano (MI) (IT)**

(72) Inventors:
- **Papeo, Gianluca Mariano Enrico**
  **23870 Cernusco Lombardone (LC) (IT)**
- **Caronni, Dannica**
  **20020 Lainate (MI) (IT)**
- **Dalvit, Claudio**
  **20154 Milan (IT)**
- **Giordano, Patrizia**
  **12100 Cuneo (IT)**
- **Mongelli, Nicola**
  **20137 Milan (IT)**
- **Veronesi, Marina**
  **20148 Milan (IT)**
- **Ciprandi, Franco**
  **20014 Nerviano (MI) (IT)**

(74) Representative: **Mazzini, Giuseppe**
**Nerviano Medical Sciences S.r.l.**
**Patent Department**
**Viale Pasteur, 10**
**20014 Nerviano (Milano) (IT)**

(56) References cited:
**WO-A-2005/005978**

- **DALVIT C ET AL.: "Rapid NMR-based functional screening and IC50 measurements performed at unprecedentedly low enzyme concentration" DRUG DEVELOPMENT RESEARCH, vol. 64, no. 2, February 2005 (2005-02), pages 105-113, XP009094341 ISSN: 0272-4391**
- **SMITS R ET AL.: "How C-alpha-fluoroalkyl amino acids and peptides interact with enzymes: Studies concerning the influence on proteolytic stability, enzymatic resolution and peptide coupling" CURRENT TOPICS IN MEDICINAL CHEMISTRY, vol. 6, no. 14, July 2006 (2006-07), pages 1483-1498, XP009096302 ISSN: 1568-0266**
- **PAPEO G ET AL.: "Polyfluorinated amino acids for sensitive 19F NMR-based screening and kinetic measurements." JOURNAL OF THE AMERICAN CHEMICAL SOCIETY, vol. 129, no. 17, 7 April 2007 (2007-04-07), pages 5665-5672, XP009094331 ISSN: 0002-7863**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 923 397 B1

SEQUENCE LISTING

<110> Nerviano Medical Sciences Srl.

<120> FLUORINATED AMINO ACIDS AND PEPTIDES

<130> NMS010

<160> 102

<170> PatentIn version 3.3

<210> 1
<211> 5
<212> PRT
<213> Artificial

<220>
<223> artificail peptide

<220>
<221> MOD_RES
<222> (2)..(2)
<223> X = X1 as defined in the specification

<220>
<221> MOD_RES
<222> (5)..(5)
<223> AMIDATION

<400> 1

Glu Xaa Ala Arg Ala
1               5

<210> 2
<211> 5
<212> PRT
<213> Artificial

<220>
<223> artificial peptide

<220>
<221> MOD_RES
<222> (2)..(2)
<223> X = X2 as defined in the specification

<220>
<221> MOD_RES
<222> (5)..(5)
<223> AMIDATION

<400> 2

Glu Xaa Ala Arg Ala
1               5

<210> 3
<211> 5
<212> PRT
<213> Artificial
<220>
<223> artificial peptide
<220>
<221> MOD_RES
<222> (2)..(2)
<223> X = X3 as defined in the specification
<220>
<221> MOD_RES
<222> (5)..(5)
<223> AMIDATION
<400> 3

Glu Xaa Ala Arg Ala
1               5

<210> 4
<211> 5
<212> PRT
<213> Artificial
<220>
<223> artificial peptide
<220>

<221> MOD_RES
<222> (2)..(2)
<223> X = X4 as defined in the specification
<220>
<221> MOD_RES
<222> (5)..(5)
<223> AMIDATION
<400> 4

```
Glu Xaa Ala Arg Ala
1               5
```

<210> 5
<211> 5
<212> PRT
<213> Artificial
<220>
<223> artificial peptide
<220>
<221> MOD_RES
<222> (2)..(2)
<223> X = X5 as defined in the specification
<220>
<221> MOD_RES
<222> (5)..(5)
<223> AMIDATION
<400> 5

```
Glu Xaa Ala Arg Ala
1               5
```

<210> 6
<211> 5
<212> PRT
<213> Artificial
<220>
<223> artificial peptide
<220>
<221> MOD_RES
<222> (2)..(2)
<223> X = X0 as defined in the specification
<220>
<221> MOD_RES
<222> (5)..(5)
<223> AMIDATION
<400> 6

```
Glu Xaa Ala Arg Ala
1               5
```

<210> 7
<211> 6
<212> PRT

<213> Artificial
<220>
<223> articial peptide
<220>
<221> MOD_RES
<222> (1)..(1)
<223> X = X0 as defined in the specification
<220>
<221> MOD_RES
<222> (6)..(6)
<223> AMIDATION
<400> 7

```
Xaa Ser Ala Val Arg Ile
1               5
```

<210> 8
<211> 6
<212> PRT
<213> Artificial
<220>
<223> Artificial peptide
<220>
<221> MOD_RES
<222> (1)..(1)
<223> x = X0 as defined in the specification
<220>
<221> MOD_RES
<222> (6)..(6)
<223> AMIDATION
<400> 8

```
Xaa Glu Ala Val Arg Ile
1               5
```

<210> 9
<211> 6
<212> PRT
<213> Artificial
<220>
<223> Artificial peptide
<220>
<221> MOD_RES
<222> (1)..(1)
<223> X = X0 as defined in the specification
<220>
<221> MOD_RES
<222> (6)..(6)
<223> AMIDATION
<400> 9

```
Xaa Ser Ala Lys Arg Ile
1               5
```

<210> 10
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 10

```
Xaa Glu Ala Lys Arg Ile
1               5
```

<210> 11
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 11

```
Xaa Ser Ala Gly Arg Ile
1               5
```

<210> 12
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES

<222> (6)..(6)
<223> AMIDATION
<400> 12

```
Xaa Glu Ala Gly Arg Ile
1               5
```

<210> 13
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 13

```
Xaa Ser Phe Val Arg Ile
1               5
```

<210> 14
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 14

```
Xaa Glu Phe Val Arg Ile
1               5
```

<210> 15
<211> 6
<212> PRT
<213> Artificial
<220>
<223> artificial peptide
<220>
<221> MOD_RES

<222> (1)..(1)
<223> X = X0 as defined in the specification
<220>
<221> MOD_RES
<222> (6)..(6)
<223> AMIDATION
<400> 15

```
Xaa Ser Phe Lys Arg Ile
1               5
```

<210> 16
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 16

```
Xaa Glu Phe Lys Arg Ile
1               5
```

<210> 17
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 17

```
Xaa Ser Phe Gly Arg Ile
1               5
```

<210> 18
<211> 6
<212> PRT

<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 18

```
Xaa Glu Phe Gly Arg Ile
1               5
```

<210> 19
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 19

```
Xaa Ser Leu Val Arg Ile
1               5
```

<210> 20
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 20

```
Xaa Glu Leu Val Arg Ile
1               5
```

<210> 21
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 21

```
Xaa Ser Leu Lys Arg Ile
1               5
```

<210> 22
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 22

```
Xaa Glu Leu Lys Arg Ile
1               5
```

<210> 23
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 23

```
Xaa Ser Leu Gly Arg Ile
1               5
```

<210> 24
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 24

```
Xaa Glu Leu Gly Arg Ile
1               5
```

<210> 25
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION
<400> 25

```
Xaa Ser Gln Val Arg Ile
1               5
```

<210> 26
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES

<222> (6)..(6)
<223> AMIDATION
<400> 26

Xaa Glu Gln Val Arg Ile
1               5

<210> 27
<211> 6
<212> PRT
<213> Artificial
<220>
<223> artificial peptide
<220>
<221> MOD_RES
<222> (1)..(1)
<223> X = X0 as defined in the specification
<220>
<221> MOD_RES
<222> (6)..(6)
<223> AMIDATION

<400> 27

Xaa Ser Gln Lys Arg Ile
1               5

<210> 28
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 28

Xaa Glu Gln Lys Arg Ile
1               5

<210> 29
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 29

```
Xaa Ser Gln Gly Arg Ile
1               5
```

<210> 30
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 30

```
Xaa Glu Gln Gly Arg Ile
1               5
```

<210> 31
<211> 6
<212> PRT
<213> Artificial

<220>
<223> artifical peptide

<220>
<221> MOD_RES
<222> (1)..(1)
<223> X = X0 as defined in the specification

<220>

<221> MOD_RES
<222> (6)..(6)
<223> AMIDATION

<400> 31

```
Xaa Arg Val Ala Glu Ile
1               5
```

<210> 32
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 32

```
Xaa Arg Val Ala Ser Ile
1               5
```

<210> 33
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 33

Xaa Arg Lys Ala Glu Ile
1 5

<210> 34
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 34

Xaa Arg Lys Ala Ser Ile
1 5

<210> 35
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 35

Xaa Arg Gly Ala Glu Ile

1 5

<210> 36
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 36

```
Xaa Arg Gly Ala Ser Ile
1               5
```

<210> 37
<211> 6
<212> PRT
<213> Artificial

<220>
<223> artifical peptide

<220>
<221> MOD_RES
<222> (1)..(1)
<223> X = X0 as defined in the specification

<220>
<221> MOD_RES
<222> (6)..(6)
<223> AMIDATION

<400> 37

```
Xaa Arg Val Phe Glu Ile
1               5
```

<210> 38
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 38

```
Xaa Arg Val Phe Ser Ile
1               5
```

<210> 39
<211> 6
<212> PRT
<213> Artificial

<220>
<223> artificial peptide

<220> .
<221> MOD_RES
<222> (1)..(1)
<223> X = X0 as defined in the specification

<220>
<221> MOD_RES
<222> (6)..(6)
<223> AMIDATION

<400> 39

```
Xaa Arg Lys Phe Glu Ile
1               5
```

<210> 40
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 40

Xaa Arg Lys Phe Ser Ile
1               5

<210> 41
<211> 6
<212> PRT
<213> Artificial

<220>
<223> artifical peptide

<220>
<221> MOD_RES
<222> (1)..(1)
<223> X = X0 as defined in the specification

<220>
<221> MOD_RES
<222> (6)..(6)
<223> AMIDATION

<400> 41

Xaa Arg Gly Phe Glu Ile
1               5

<210> 42
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 42

Xaa Arg Gly Phe Ser Ile
1               5

<210> 43
<211> 6
<212> PRT

<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 43

```
Xaa Arg Val Leu Glu Ile
1               5
```

<210> 44
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 44

```
Xaa Arg Val Leu Ser Ile
1               5
```

<210> 45
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 45

```
Xaa Arg Lys Leu Glu Ile
1               5
```

<210> 46
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 46

```
Xaa Arg Lys Leu Ser Ile
1               5
```

<210> 47
<211> 6
<212> PRT
<213> Artificial

<220>
<223> artificail peptide

<220>
<221> MOD_RES
<222> (1)..(1)
<223> X = X0 as defined in the specification

<220>
<221> MOD_RES
<222> (6)..(6)
<223> AMIDATION

<400> 47

Xaa Arg Gly Leu Glu Ile
1 5

<210> 48
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 48

Xaa Arg Gly Leu Ser Ile
1 5

<210> 49
<211> 6
<212> PRT
<213> Artificial

<220>
<223> artifical peptide

<220>
<221> MOD_RES
<222> (1)..(1)
<223> X = X0 as defined in the specification

<220>
<221> MOD_RES
<222> (6)..(6)
<223> AMIDATION

<400> 49

Xaa Arg Val Gln Glu Ile
1 5

<210> 50
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 50

```
Xaa Arg Val Gln Ser Ile
1               5
```

<210> 51
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 51

```
Xaa Arg Lys Gln Glu Ile
1               5
```

<210> 52
<211> 6
<212> PRT
<213> Artificial

<220>
<223> artifical peptide

<220>
<221> MOD_RES
<222> (1)..(1)
<223> X = X0 as defined in the specification

<220>

<221> MOD_RES
<222> (6)..(6)
<223> AMIDATION

<400> 52

```
Xaa Arg Lys Gln Ser Ile
1               5
```

<210> 53
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 53

```
Xaa Arg Gly Gln Glu Ile
1               5
```

<210> 54
<211> 6
<212> PRT
<213> Artificial

<220>
<223> artificial peptide

<220>
<221> MOD_RES
<222> (1)..(1)
<223> X = X0 as defined in the specification

<220>
<221> MOD_RES
<222> (6)..(6)
<223> AMIDATION

<400> 54

```
Xaa Arg Gly Gln Ser Ile
1               5
```

<210> 55
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 55

```
Xaa Ala Asp Arg Val Ser Ile
1               5
```

<210> 56
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 56

```
Xaa Ala Glu Arg Val Ser Ile
1               5
```

<210> 57
<211> 7
<212> PRT
<213> Artificial

<220>
<223> artificial peptide

<220>
<221> MOD_RES
<222> (1)..(1)
<223> x = x0 as defined in the specification

<220>
<221> MOD_RES
<222> (7)..(7)
<223> AMIDATION

<400> 57

```
Xaa Ala Asp Arg Lys Ser Ile
1               5
```

<210> 58
<211> 7
<212> PRT
<213> Artificial

<220>
<223> artificial peptideù

<220>
<221> MOD_RES
<222> (1)..(1)
<223> X = X0 as defined in the specification

<220>
<221> MOD_RES
<222> (7) .. (7)
<223> AMIDATION

<400> 58

```
Xaa Ala Glu Arg Lys Ser Ile
1               5
```

<210> 59
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 59

```
Xaa Ala Asp Arg Gly Ser Ile
1               5
```

<210> 60
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 60

```
Xaa Ala Glu Arg Gly Ser Ile
1               5
```

<210> 61
<211> 7
<212> PRT
<213> Artificial

<220>
<223> artifical peptide

<220>
<221> MOD_RES
<222> (1)..(1)
<223> X = X0 as defined in the specification

<220>
<221> MOD_RES
<222> (7)..(7)
<223> AMIDATION

<400> 61

```
Xaa Phe Asp Arg Val Ser Ile
1               5
```

<210> 62
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 62

```
Xaa Phe Glu Arg Val Ser Ile
1               5
```

<210> 63
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 63

```
Xaa Phe Asp Arg Lys Ser Ile
1               5
```

<210> 64
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 64

```
Xaa Phe Glu Arg Lys Ser Ile
1               5
```

<210> 65
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 65

```
Xaa Phe Asp Arg Gly Ser Ile
1               5
```

<210> 66
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES

<222> (7)..(7)
<223> AMIDATION

<400> 66

```
Xaa Phe Glu Arg Gly Ser Ile
1               5
```

<210> 67
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 67

```
Xaa Leu Asp Arg Val Ser Ile
1               5
```

<210> 68
<211> 7
<212> PRT
<213> Artificial

<220>
<223> artifcial peptide

<220>
<221> MOD_RES
<222> (1)..(1)
<223> X = X0 as defined in the specification

<220>
<221> MOD_RES
<222> (7)..(7)
<223> AMIDATION

<400> 68

```
Xaa Leu Glu Arg Val Ser Ile
1               5
```

<210> 69
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 69

```
Xaa Leu Asp Arg Lys Ser Ile
1               5
```

<210> 70
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 70

```
Xaa Leu Glu Arg Lys Ser Ile
1               5
```

<210> 71
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 71

```
Xaa Leu Asp Arg Gly Ser Ile
1               5
```

<210> 72
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 72

```
Xaa Leu Glu Arg Gly Ser Ile
1               5
```

<210> 73
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)

<223> AMIDATION

<400> 73

```
Xaa Gln Asp Arg Val Ser Ile
1               5
```

<210> 74
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 74

```
Xaa Gln Glu Arg Val Ser Ile
1               5
```

<210> 75
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 75

```
Xaa Gln Asp Arg Lys Ser Ile
1               5
```

<210> 76
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 76

```
Xaa Gln Glu Arg Lys Ser Ile
1               5
```

<210> 77
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 77

```
Xaa Gln Asp Arg Gly Ser Ile
1               5
```

<210> 78
<211> 7
<212> PRT
<213> Artificial

<220>
<223> artificial peptide

<220>

<221> MOD_RES
<222> (1)..(1)
<223> X = X0 as defined in the specification

<220>
<221> MOD_RES
<222> (7)..(7)
<223> AMIDATION

<400> 78

```
Xaa Gln Glu Arg Gly Ser Ile
1               5
```

<210> 79
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 79

```
Xaa Val Arg Asp Ala Ser Ile
1               5
```

<210> 80
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 80

Xaa Val Arg Glu Ala Ser Ile
1               5

<210> 81
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 81

Xaa Lys Arg Asp Ala Ser Ile
1               5

<210> 82
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 82

Xaa Lys Arg Glu Ala Ser Ile
1               5

<210> 83

<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 83

```
Xaa Gly Arg Asp Ala Ser Ile
1               5
```

<210> 84
<211> 7
<212> PRT
<213> Artificial

<220>
<223> artifical peptide

<220>
<221> MOD_RES
<222> (1)..(1)
<223> X = X0 as defined in the specification

<220>
<221> MOD_RES
<222> (7)..(7)
<223> AMIDATION

<400> 84

```
Xaa Gly Arg Glu Ala Ser Ile
1               5
```

<210> 85
<211> 7
<212> PRT
<213> Artificial

<220>
<223> artifical peptide

<220>
<221> MOD_RES

<222> (1)..(1)
<223> X = X0 as defined in the specification

<220>
<221> MOD_RES
<222> (7)..(7)
<223> AMIDATION

<400> 85

```
Xaa Val Arg Asp Phe Ser Ile
1               5
```

<210> 86
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 86

```
Xaa Val Arg Glu Phe Ser Ile
1               5
```

<210> 87
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 87

Xaa Lys Arg Asp Phe Ser Ile
1               5

<210> 88
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 88

Xaa Lys Arg Glu Phe Ser Ile
1               5

<210> 89
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 89

Xaa Gly Arg Asp Phe Ser Ile
1               5

<210> 90

<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 90

```
Xaa Gly Arg Glu Phe Ser Ile
1               5
```

<210> 91
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 91

```
Xaa Val Arg Asp Leu Ser Ile
1               5
```

<210> 92
<211> 7
<212> PRT
<213> Artificial

<220>
<223> artificial peptide

<220>
<221> MOD_RES

<222> (1)..(1)
<223> X = X0 as defined in the specification

<220>
<221> MOD_RES
<222> (7)..(7)
<223> AMIDATION

<400> 92

```
Xaa Val Arg Glu Leu Ser Ile
1               5
```

<210> 93
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 93

```
Xaa Lys Arg Asp Leu Ser Ile
1               5
```

<210> 94
<211> 7
<212> PRT
<213> Artificial

<220>
<223> artificial peptide

<220>
<221> MOD_RES
<222> (1)..(1)
<223> x = X0 as defined in the specification

<220>
<221> MOD_RES
<222> (7)..(7)
<223> AMIDATION

<400> 94

Xaa Lys Arg Glu Leu Ser Ile
1               5

<210> 95
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 95

Xaa Gly Arg Asp Leu Ser Ile
1               5

<210> 96
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 96

Xaa Gly Arg Glu Leu Ser Ile
1               5

<210> 97
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 97

```
Xaa Val Arg Asp Gln Ser Ile
1               5
```

<210> 98
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 98

```
Xaa Val Arg Glu Gln Ser Ile
1               5
```

<210> 99
<211> 7
<212> PRT
<213> Artificial

<220>
<223> artificial peptide

<220>
<221> MOD_RES
<222> (1)..(1)
<223> X = X0 as defined in the specification

<220>

<221> MOD_RES
<222> (7)..(7)
<223> AMIDATION

<400> 99

```
Xaa Lys Arg Asp Gln Ser Ile
1               5
```

<210> 100
<211> 7
<212> PRT
<213> Artificial

<220>
<223> articial peptide

<220>
<221> MOD_RES
<222> (1)..(1)
<223> X = X0 as defined in the specification

<220>
<221> MOD_RES
<222> (7)..(7)
<223> AMIDATION

<400> 100

```
Xaa Lys Arg Glu Gln Ser Ile
1               5
```

<210> 101
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 101

```
Xaa Gly Arg Asp Gln Ser Ile
1               5
```

<210> 102
<211> 7
<212> PRT
<213> Artificial

<220>
<223> artificial peptide

<220>
<221> MOD_RES
<222> (1)..(1)
<223> X = X0 as defined in the specification

<220>
<221> MOD_RES
<222> (7)..(7)
<223> AMIDATION

<400> 102

```
Xaa Gly Arg Glu Gln Ser Ile
1               5
```

## Description

### Field of the Invention

The present invention relates to novel fluorinated amino acids and peptide chains incorporating them, to a process for their preparation, and to their use in $^{19}F$ NMR screening for bioactive compounds.

### Discussion of the Background

The testing of a large number of molecules in an appropriate biochemical or cellular assay is usually one of the first steps in a drug discovery project. Screening based on Fluorescence Polarization (FP), Fluorescence Resonance Energy Transfer (FRET), Homogeneous Time-Resolved Fluorescence (HTRF) and the traditional Scintillation Proximity Assay (SPA) are nowadays the technologies of choice for functional assays.

The sensitivity and miniaturized format of these methodologies allow for the high throughput screening (HTS) of large proprietary compound collections. However, the complexity of the assays utilized in HTS often results in a large number of hits not confirmed in a subsequent secondary assay. It is now well accepted that for screening quality is more important than quantity. Therefore, more reliable ways of performing the assays for the identification of lead molecules are constantly needed. Over the last few years Nuclear Magnetic Resonance (NMR)-based screening has emerged as a powerful tool for lead molecules identification. Two of these methodologies utilize $^{19}F$ NMR spectroscopy. The first one, FAXS (Fluorine chemical shift Anisotropy and eXchange for Screening) is a binding assay, while the second one, 3-FABS (three Fluorine Atoms for Biochemical Screening) is a functional assay. Their applications in screening are described and claimed in the published patent applications W02004/051214 and WO2005/0005978.

The results of studies on the interaction of C-alfa-fluoroalkyl amino acids and peptides with target proteins or receptors are described and discussed in the review of Smits, R et al, Current Topics in Medicinal Chemistry vol. 6, no. 14, July 2006, pages 1483-1498.

Fluorine-containing peptides represent valuable tools for $^{19}F$ NMR-based screening for new enzyme inhibitors. To work properly, these peptides must first behave as substrates for the targeted enzyme. Secondly, $^{19}F$ NMR isotropic chemical shifts of the starting peptides and of the corresponding products of the enzymatic reactions need to be appreciable different. Based on the simple principle that the more magnetically equivalent fluorine atoms are present in the peptide, the less amount of substrate (and of enzyme) is required, PFG (Poly-Fluorinated Glycine of formula A) was synthesized:

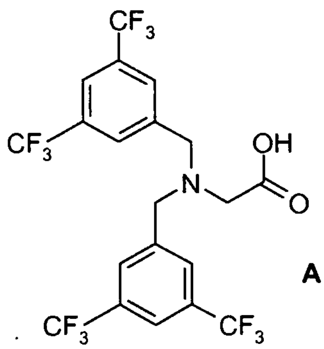

A

and used to cap different peptides [see Dalvit, Claudio et al., "Rapid NMR-based functional screening and IC50 measurements performed at unprecedentedly low enzyme concentration", Drug Development Research 2005, 64, 105-113].

Small PFG-containing peptides were successfully tested against Trypsin, and then the synthetic efforts were focused towards the preparation of an effective fluorine-containing AKT substrate. However, the systematic elongation of a previously prepared peptide unveiled the limits of PFG. The shorter sequence that turned out to be a good substrate for AKT has in fact a seven-residue distance between the site of phosphorylation and PFG. In that case, this distance appeared to be critical in terms of substrate/product chemical shifts separation. One drawback of PFG lies on the positioning of this amino acid at the N-terminus of a given peptide. To overcome this problem, we developed useful and more versatile PFG-like amino acids that can also be placed along the backbone of the peptide, if necessary.

Accordingly, the present invention provides poly-fluorinated amino acids, armed with 12 (4 X CF;) and 6 (2 X CF;) fluorine atoms. The incorporation into small peptides through solid phase peptide synthesis (SPPS), as well as the peptides so obtained and their use in $^{19}F$ NMR screening for bioactive compounds are also provided by the present

invention.

**Summary of the Invention**

The present invention provides a compound of the formula (I):

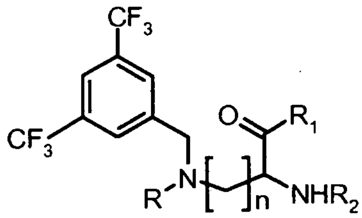

**(I)**

wherein R is hydrogen atom, a N-protecting group or 3,5-bis(trifluoromethyl)benzyl group; $R_1$ is hydroxy, $C_1$-$C_4$ alkyl group, $C_1$-$C_4$ alkoxy or aryl $C_1$-$C_4$ alkyloxy group, or the N terminus of any α-amino acid or peptide sequence; $R_2$ is hydrogen atom, a N-protecting group or the C terminus of any α-amino acid or peptide sequence; n is 1, 2, 3 or 4, or a salt thereof.

Accordingly, in a second embodiment, the present invention provides a method of using the compounds of formula (I) for detecting through "Fluorine chemical shift Anisotropy and eXchange for Screening" (FAXS) experiments weak binders, for identifying inhibitors of an enzymatic reaction or for identifying the most efficient substrate for an enzyme by means of "three Fluorine Atoms for Biochemical Screening" (3-FABS) method. The present invention also includes methods for synthesizing compounds of formula (I) and their salts. A more complete appreciation of the invention and many of the attendant advantages thereof will be readily obtained as the same becomes better understood by reference to the following detailed description.

## DETAILED DESCRIPTION OF THE INVENTION

The compounds of formula (I) of the invention have asymmetric carbon atoms and may therefore exist as individual optical isomers, as racemic mixtures or as any other mixture comprising a majority of one of the two optical isomers, which are all to be intended as within the scope of the present invention.

In the present description, unless otherwise specified, with the term N-protecting group we intend any group usually employed in the solid and the liquid phase peptides synthesis for the protection of the N-terminus of the amino acids. This N-terminus may be, for instance, optionally substituted, especially by halo-groups, acyl groups, e.g. acetyl, dichloroacetyl, monochloroacetyl, trifluoroacetyl, benzoyl or p-bromophenacyl; triarylmethylgroups, e.g. triphenylmethyl; silyl groups, in particular trimethylsilyl, dimethyl-*t*-butylsilyl, diphenyl- *t*-butylsilyl; or also groups such as 9-fluorenylmethoxycarbonyl, benzyloxycarbonyl, *t*-butoxycarbonyl, p-nitrobenzyloxycarbonyl, 2,2,2-trichloroethoxycarbonyl, benzyl and pyranyl.

With the term $C_1$-$C_4$ alkyl or $C_1$-$C_4$ alkoxy we intend any of the straight or branched groups such as methyl, ethyl, *n*-propyl, isopropyl, butyl, *i*-butyl, t-butyl, *sec*-butyl, methoxy, ethoxy, *n*-propoxy, isopropoxy, *n*-butoxy, iso-butoxy and *t*-butoxy.

With the term aryl $C_1$-$C_4$ alkyloxy group we intend a group such as benzyloxy, triphenylmethoxy, diphenylmethoxy, bis(o-nitrophenyl)methoxy, 9-antrhylmethoxy, 2,4,6-trimethylbenzyloxy, p-bromobenzyloxy, o-nitrobenzyloxy, p-nitrobenzyloxy, p-methoxybenzyloxy, 2,6-dimethoxybenzyloxy, 4-(methylsulfinyl)benzyloxy, 4-sulfobenzyloxy.

With the term halogen atom we intend a fluorine, chlorine, bromine or iodine atom.

With the term α-amino acid we intend any of the naturally and unnaturally occurring α-amino acid, such as, for example, Alanine, alpha-Aminobutyric acid, alpha-Aminoisobutyric acid, Arginine, Asparagine, Aspartic acid, tert-Butylglycine, Citrulline, Cyclohexylalanine, Cysteine, Glutamic acid, Glutamine, Glycine, Histidine, Homoserine, Hydroxyproline, Isoleucine, Leucine, Lysine, ter-Leucine, Methionine, Norleucine, Norvaline, Ornitine, Phenylalanine, Phenylglycine, Proline, Sarcosine, Serine, Threonine, Triptophan, Tyrosine, Valine. All α-amino acids in both L and D configuration are intended to be enclosed in the present invention.

With the term peptide sequence we intend a sequence formed by any α-amino acid, such as those indicated in the Table 1, where the single letter code is used.

The compounds of the invention are chemically stable in solution at neutral pH, and can form salts by acid or base addition depending on the nature of the peptide. All these forms are intended to be enclosed in the present invention,

preferably, salts with sodium, potassium and ammonium cationic counterpart and salts with chloride, formate, acetate and trifluoroacetate anionic counterpart.

All of these salts may be prepared by conventional means from the corresponding compounds of the present invention, for instance by reacting them with the appropriate acid or base. A preferred class of compounds of the invention is represented by the derivatives of formula (I) wherein:

R is hydrogen atom, 3,5-bis(trifluoromethyl)benzyl group, t-butoxycarbonyl group, 9-fluorenylmethoxycarbonyl group;
$R_1$ is hydroxy or the N terminus of any α-amino acid or peptide sequence; $R_2$ is hydrogen atom, t-butoxycarbonyl group, 9-fluorenylmethoxycarbonyl group or the C terminus of any α-amino acid or peptide sequence and n is 2, 3 or 4.

According to a preferred embodiment, within the compounds of formula (I), R is hydrogen atom or 3,5-bis (trifluoromethyl)benzyl group, $R_1$ is hydroxy, $R_2$ is 9-fluorenylmethoxycarbonyl.

According to another preferred embodiment, within the compounds of formula (I), R is *t*-butoxycarbonyl; $R_1$ is hydroxy, $R_2$ is 9-fluorenylmethoxycarbonyl.

According to a further preferred embodiment, within the compounds of formula (I), R is 3,5-bis(trifluoromethyl) benzyl group, $R_1$ is hydroxy, $R_2$ is 9-fluorenylmethoxycarbonyl.

It is to be noted that compounds within the scope of the present invention characterized by having only two $CF_3$- groups (R=H) gave rise to better signal separation and to more soluble peptides in aqueous medium. Therefore, even more preferably, R is hydrogen atom, t-butoxycarbonyl or 9-fluorenylmethoxycarbonyl group. Further preferred compounds of the formula (I) are those wherein $R_1$ is the N terminus of any α-amino acid or peptide sequence, and $R_2$ is the C terminus of any α-amino acid or peptide sequence, or a salt thereof. Other preferred compounds of the formula (I) are those wherein $R_1$ is the N terminus of a peptide sequence, and $R_2$ is the C terminus of any α-amino acid or hydrogen atom.

Representative compounds that are within the scope of the present invention are reported in Table 1, wherein in the peptides of entries 15-20, $R_1$ is the N terminus of a peptide sequence, and $R_2$ is the C terminus of an α-amino acid, and in the peptides of entries 21-116, $R_1$ is the N terminus of a peptide sequence, and $R_2$ is hydrogen atom:

Table 1

| Entry | Sequence | [SEQ ID NO] |
|---|---|---|
| **15** | $EX_1$ ARA-$NH_2$ | [SEQ ID NO.:1] |
| **16** | $EX_2$ ARA-$NH_2$ | [SEQ ID NO.:2] |
| **17** | E $X_3$ A R A-$NH_2$ | [SEQ ID NO.:3] |
| **18** | E $X_4$ A R A-$NH_2$ | [SEQ ID NO.:4] |
| **19** | E $X_5$ A R A-$NH_2$ | [SEQ ID NO.:5] |
| **20** | E $X_0$ A R A-$NH_2$ | [SEQ ID NO.:6] |
| **21** | $X_0$ S A V R I-$NH_2$ | [SEQ ID NO.:7] |
| **22** | $X_0$ E A V R I-$NH_2$ | [SEQ ID NO.:8] |
| **23** | $X_0$ S A K R I-$NH_2$ | [SEQ ID NO.:9] |
| **24** | $X_0$ E A K R I-$NH_2$ | [SEQ ID NO.:10] |
| **25** | $X_0$ S A G R I-$NH_2$ | [SEQ ID NO.:11] |
| **26** | $X_0$ E A G R I-$NH_2$ | [SEQ ID NO.:12] |
| **27** | $X_0$ S F V R I-$NH_2$ | [SEQ ID NO.:13] |
| **28** | $X_0$ EFVRI-$NH_2$ | [SEQ ID NO.:14] |
| **29** | $X_0$ S F K R I-$NH_2$ | [SEQ ID NO.:15] |
| **30** | $X_0$ E F K R I-$NH_2$ | [SEQ ID NO.:16] |
| **31** | $X_0$ S F G R I-$NH_2$ | [SEQ ID NO.:17] |
| **32** | $X_0$ E F G R I-$NH_2$ | [SEQ ID NO.:18] |
| **33** | $X_0$ S L V R I-$NH_2$ | [SEQ ID NO.:19] |

(continued)

| Entry | Sequence | [SEQ ID NO] |
|---|---|---|
| **34** | $X_0$ E L V R I-$NH_2$ | [SEQ ID NO.:20] |
| **35** | $X_0$ S L K R I-$NH_2$ | [SEQ ID NO.:21] |
| **36** | $X_0$ E L K R I-$NH_2$ | [SEQ ID NO.:22] |
| **37** | $X_0$ S L G R I-$NH_2$ | [SEQIDNO.:23] |
| **38** | $X_0$ E L G R I-$NH_2$ | [SEQ ID NO.:24] |
| **39** | $X_0$ S Q V R I-$NH_2$ | [SEQ ID NO.:25] |
| **40** | $X_0$ E Q V R I-$NH_2$ | [SEQ ID NO.:26] |
| **41** | $X_0$ S Q K R I-$NH_2$ | [SEQ ID NO.:27] |
| **42** | $X_0$ E Q K R I-$NH_2$ | [SEQ ID NO.:28] |
| **43** | $X_0$ S Q G R I-$NH_2$ | [SEQ ID NO.:29] |
| **44** | $X_0$ E Q G R I-$NH_2$ | [SEQ ID NO.:30] |
| **45** | $X_0$ R V A E I-$NH_2$ | [SEQIDNO.:31] |
| **46** | $X_0$ R V A S I-$NH_2$ | [SEQ ID NO.:32] |
| **47** | $X_0$ R K A E I-$NH_2$ | [SEQ ID NO.:33] |
| **48** | $X_0$ R K A S I-$NH_2$ | [SEQ ID NO.:34] |
| **49** | $X_0$ R G A E I-$NH_2$ | [SEQ ID NO.:35] |
| **50** | $X_0$ R G A S I-$NH_2$ | [SEQ ID NO.:36] |
| **51** | $X_0$ R V F E I-$NH_2$ | [SEQ ID NO.:37] |
| **52** | $X_0$ R V F S I-$NH_2$ | [SEQ ID NO.:38] |
| **53** | $X_0$ R K F E I-$NH_2$. | [SEQ ID NO.:39] |
| **54** | $X_0$ R K F S I-$NH_2$ | [SEQ ID NO.:40] |
| **55** | $X_0$ R G F E I-$NH_2$ | [SEQ ID NO.:41] |
| **56** | $X_0$ R G F S I-$NH_2$ | [SEQ ID NO.:42] |
| **57** | $X_0$ R V L E I-$NH_2$ | [SEQ ID NO.:43] |
| **58** | $X_0$ R V L S I-$NH_2$ | [SEQ ID NO.:44] |
| **59** | $X_0$ R K L E I-NH, | [SEQ ID NO.:45] |
| **60** | $X_0$ R K L S I-$NH_2$ | [SEQ ID NO.:46] |
| **61** | $X_0$ R G L E I-$NH_2$ | [SEQ ID NO.:47] |
| **62** | $X_0$ R G L S I-$NH_2$ | [SEQ ID NO.:48] |
| **63** | $X_0$ R V Q E I-$NH_2$ | [SEQ ID NO.:49] |
| **64** | $X_0$ R V Q S I-$NH_2$ | [SEQ ID NO.:50] |
| **65** | $X_0$ R K Q E I-$NH_2$ | [SEQ ID NO.:51] |
| **66** | $X_0$ R K Q S I-$NH_2$ | [SEQ ID NO.:52] |
| **67** | $X_0$ R G Q E I-$NH_2$ | [SEQ ID NO.:53] |
| **68** | $X_0$ R G Q S I-$NH_2$ | [SEQ ID NO.:54] |
| **69** | $X_0$ A D R V S I-$NH_2$ | [SEQ ID NO.:55] |
| **70** | $X_0$ A E R V S I-$NH_2$ | [SEQ ID NO.:56] |
| **71** | $X_0$ A D R K S I-$NH_2$ | [SEQ ID NO.:57] |

(continued)

| Entry | Sequence | [SEQ ID NO] |
|---|---|---|
| **72** | $X_0$ A E R K S I-$NH_2$ | [SEQ ID NO.:58] |
| **73** | $X_0$ A D R G S I-$NH_2$ | [SEQ ID NO.:59] |
| **74** | $X_0$ A E R G S I-$NH_2$ | [SEQ ID NO.:50] |
| **75** | $X_0$ F D R V S I-$NH_2$ | [SEQ ID NO.:61] |
| **76** | $X_0$ F E R V S I-$NH_2$ | [SEQ ID NO.:62] |
| **77** | $X_0$ F D R K S I-$NH_2$ | [SEQ ID NO.:63] |
| **78** | $X_0$ F E R K S I-$NH_2$ | [SEQ ID NO.:64] |
| **79** | $X_0$ F D R G S I-$NH_2$ | [SEQ ID NO.:65] |
| **80** | $X_0$ F E R G S I-$NH_2$ | [SEQ ID NO.:66] |
| **81** | $X_0$ L D R V S I-$NH_2$ | [SEQ ID NO.:67] |
| **82** | $X_0$ L E R V S I-$NH_2$ | [SEQ ID NO.:68] |
| **83** | $X_0$ L D R K S I-$NH_2$ | [SEQ ID NO.:69] |
| **84** | $X_0$ L E R K S I-$NH_2$ | [SEQ ID NO.:70] |
| **85** | $X_0$ L D R G S I-$NH_2$ | [SEQ ID NO.:71] |
| **86** | $X_0$ L E R G S I-$NH_2$ | [SEQ ID NO.:72] |
| **87** | $X_0$ Q D R V S I-$NH_2$ | [SEQ ID NO.:73] |
| **88** | $X_0$ Q E R V S I-$NH_2$ | [SEQ ID NO.:74] |
| **89** | $X_0$ Q D R K S I-$NH_2$ | [SEQ ID NO.:75] |
| **90** | $X_0$ Q E R K S I-$NH_2$ | [SEQ ID NO.:76] |
| **91** | $X_0$ Q D R G S I-$NH_2$ | [SEQ ID NO.:77] |
| **92** | $X_0$ Q E R G S I-$NH_2$ | [SEQ ID NO.:78] |
| **93** | $X_0$ V R D A S I-$NH_2$ | [SEQ ID NO.:79] |
| **94** | $X_0$ V R E A S I-$NH_2$ | [SEQ ID NO.:80] |
| **95** | $X_0$ K R D A S I-$NH_2$ | [SEQ ID NO.:81] |
| **96** | $X_0$ K R E A S I-$NH_2$ | [SEQ ID NO.:82] |
| **97** | $X_0$ G R D A S I-$NH_2$ | [SEQ ID NO.:83] |
| **98** | $X_0$ G R E A S I-$NH_2$ | [SEQ ID NO.:84] |
| **99** | $X_0$ V R D F S I-$NH_2$ | [SEQ ID NO.:85] |
| **100** | $X_0$ V R E F S I-$NH_2$ | [SEQ ID NO.:86] |
| **101** | $X_0$ K R D F S I-$NH_2$ | [SEQ ID NO.:87] |
| **102** | $X_0$ K R E F S I-$NH_2$ | [SEQ ID NO.:88] |
| **103** | $X_0$ G R D F S I-$NH_2$ | [SEQ ID NO.:89] |
| **104** | $X_0$ G R E F S I-$NH_2$ | [SEQ ID NO.:90] |
| **105** | $X_0$ V R D L S I-$NH_2$ | [SEQ ID NO.:91] |
| **106** | $X_0$ V R E L S I-$NH_2$ | [SEQ ID.NO.:92] |
| **107** | $X_0$ K R D L S I-$NH_2$ | [SEQ ID NO.:93] |
| **108** | $X_0$ K R E L S I-$NH_2$ | [SEQ ID NO.:94] |
| **109** | $X_0$ G R D L S I-$NH_2$ | [SEQ ID NO.:95] |

(continued)

| Entry | Sequence | [SEQ ID NO] |
|---|---|---|
| **110** | $X_0$ G R E L S I-$NH_2$ | [SEQ ID NO.:96] |
| **111** | $X_0$ V R D Q S I-$NH_2$ | [SEQ ID NO.:97] |
| **112** | $X_0$ V R E Q S I-$NH_2$ | [SEQ ID NO.:98] |
| **113** | $X_0$ K R D Q S I-$NH_2$ | [SEQ ID NO.:99] |
| **114** | $X_0$ K R E Q S I-$NH_2$ | [SEQ ID NO.:100] |
| **115** | $X_0$ G R D Q S I-$NH_2$ | [SEQ ID NO.:101] |
| **116** | $X_0$ G R E Q S I-$NH_2$ | [SEQ ID NO.:102] |

wherein $X_1$, $X_2$, $X_3$, $X_4$, $X_5$ and $X_0$, represent a fluorinated amino acid of the formula (Ia):

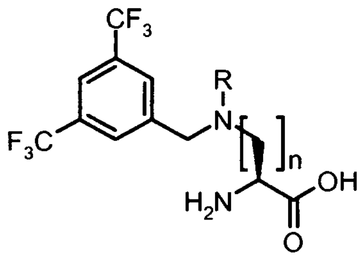

**(Ia)**

$X_1$: R = 3,5-bis(trifluoromethyl)benzyl; n = 2
$X_2$: R = 3,5-bis(trifluoromethyl)benzyl; n = 3
$X_3$: R = 3,5-bis(trifluoromethyl)benzyl; n = 4
$X_4$: R = H; n = 2
$X_5$: R = H; n = 3
$X_0$: R = H; n = 4

As stated above, any specific compound of formula (I) of the invention may be optionally in the form of a salt.

As formerly indicated, a further object of the present invention is represented by a process for preparing the compounds of formula (I) and salts thereof, as depicted in Scheme 1, wherein R, $R_1$, $R_2$ and n are as defined above unless otherwise indicated, and A- is an anion such as Cl ' or $CF_3COO$ -:

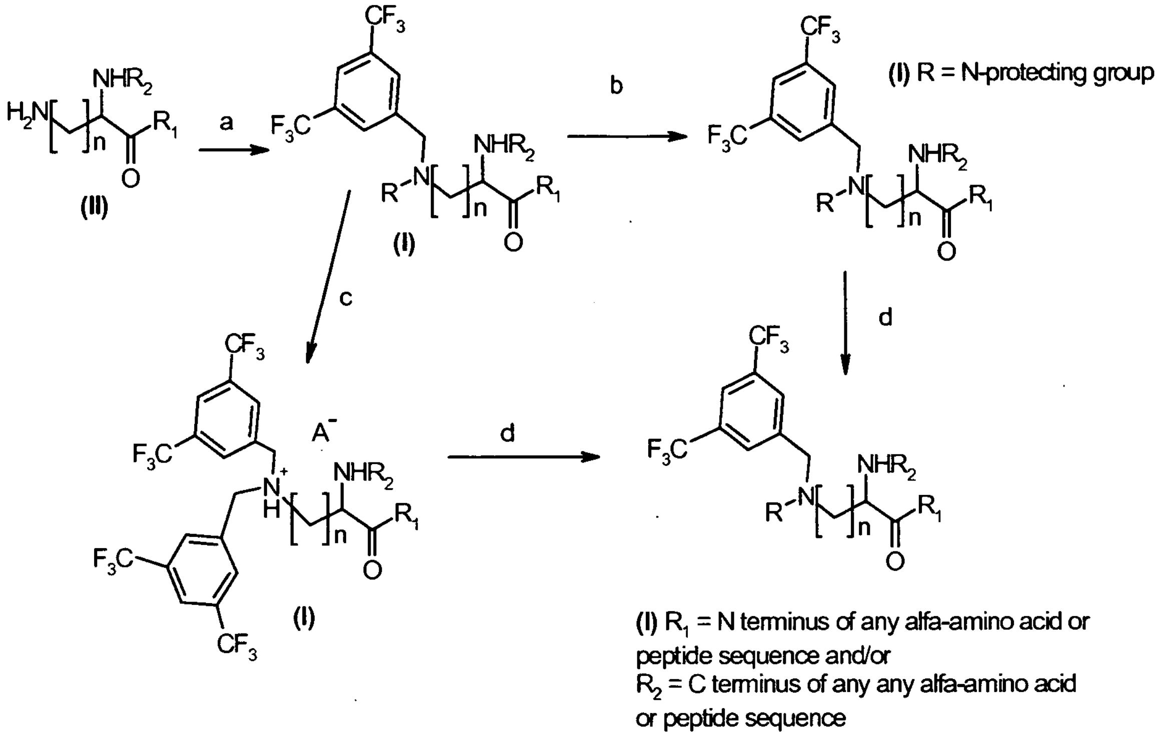


Scheme 1

Accordingly, the present invention also provides a process for preparing a compound of formua (I) as above defined, which process comprises:

a) converting a compound of the formula (II):

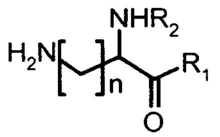 (II)

wherein $R_1$ is hydroxy, $R_2$ is a N-protecting group and n is as defined above into a corresponding compound of the formula (I) wherein R is as defined above, $R_1$ is hydroxy, $R_2$ is a N-protecting group and n is as defined above, through reductive amination with the properly choosen aldehyde and a reducing agent or alkylation with the corresponding benzyl halide in the presence of a base and either

b) installing a suitable orthogonal protective group on a resultant compound of the formula (I) when R is hydrogen atom so as to obtain a compound of the formula (I) wherein R is a N-protecting group; or

c) salifying a resultant compound of the formula (I) when R is 3,5-bis(trifluoromethyl)benzyl group with the appropriate acidic counterpart and

d) condensing the resultant compounds of the formula (I) with suitable protected amino acids, aminoacid derivatives or peptides, in the order of the amino acids of the desired final compound of the formula (I), either by liquid phase methodology or solid phase synthesis, operating by standard strategies and conditions, so as to obtain another compound of the formula (I) wherein either $R_1$ is the N terminus of any $\alpha$-amino acid or peptide sequence, or $R_2$ is the C terminus of any $\alpha$-amino acid or peptide sequence, and optionally converting the resulting compound of the formula (I) into a salt thereof.

According to step (a) of the process, compounds of the formula (I) wherein R is hydrogen atom or 3,5-bis (trifluoromethyl)benzyl group, $R_1$ is hydroxy, $R_2$ is a N-protecting group, preferably 9-fluorenylmethoxycarbonyl, and n is as defined above were prepared by reacting compounds of formula (II) as above defined with 3,5-bis(trifluoromethyl)benzaldehyde in the presence of sodium cyanoborohydride in a mixture of methanol and acetic acid at a temperature ranging from. 0 °C to room temperature and for a time varying from 5 to 24 hours or, alternatively, with sodium triacetoxyborohydride in a mixture of DMF and trifluoroacetic acid, at a temperature ranging from 0 °C to room temperature and for a time varying from 1 to 4 hours.

Preferably, step (a) is carried out by reacting a compound of formula (II) as defined above with 3,5-bis(trifluoromethyl)benzaldehyde in the presence of sodium triacetoxyborohydride in a mixture of DMF and trifluoroacetic acid, at room temperature and for a time varying from 1 to 4 hours.

The obtained compound of the formula (I) can be optionally purified before being processed in the next steps.

According to step (b) of the process, compounds of formula (I) wherein R is is a N-protecting group, preferably t-butoxycarbonyl or 9-fluorenylmethoxycarbonyl group, $R_1$ is hydroxy, $R_2$ is a N-protecting group, preferably 9-fluorenylmethoxycarbonyl or t-butoxycarbonyl and n is as defined above were prepared by reacting compounds of formula (I) wherein R is hydrogen atom, $R_1$ is hydroxy, $R_2$ is a N-protecting group, preferably 9-fluorenylmethoxycarbonyl or t-butoxycarbonyl and n is as defined above with a suitable reagent in appropriate conditions, preferably with 9-fluorenylmethyl chloroformate in the presence of diisopropylethylamine in dichloromethane at a temperature ranging from 0 °C to room temperature and for a time varying from 5 to 24 hours or with with di-tert-butyldicarbonate in the presence of saturated aqueous sodium hydrogen carbonate in ethyl acetate or dichloromethane at a temperature ranging from 0 °C to room temperature and for a time varying from 3 to 24 hours.

More preferably, step (b) is carried out by reacting compounds of formula (I) wherein R is hydrogen atom, $R_1$ is hydroxy, $R_2$ is 9-fluorenylmethoxycarbonyl and n is as defined above with di-tent-butyldicarbonate in the presence of saturated aqueous sodium hydrogen carbonate in dichloromethane at room temperature for 3 hours.

The obtained compounds of the formula (I) can be optionally purified before being processed in the next steps.

According to step (c) of the process, acid addition salts like trifluoroacetic acid or hydrochloric acid salts of compounds of formula (I) wherein R is 3,5-bis(trifluoromethyl)benzyl group, $R_1$ is hydroxy, $R_2$ is a N-protecting group, preferably 9-fluorenylmethoxycarbonyl and n is as defined above were prepared by reacting compounds of formula (I) wherein R is 3,5-bis(trifluoromethyl)benzyl group, $R_1$ is hydroxy, $R_2$ is a N-protecting group, preferably 9-fluorenylmethoxycarbonyl and n is as defined above with an acid in a appropriate solvent, preferably with trifluoroacetic acid in dichloromethane at a temperature ranging from 0 °C to room temperature and for a time varying from 1 to 24 hours, or with 4N hydrochloric acid in dioxane at a temperature ranging from 0 °C to room temperature and for a time varying from 1 to 24 hours.

More preferably, step (c) is carried out by reacting compounds of formula (I) wherein R is 3,5-bis(trifluoromethyl) benzyl group; $R_1$ is hydroxy, $R_2$ is a N-protecting group, preferably 9-fluorenylmethoxycarbonyl and n is as defined above with 4N hydrochloric acid in dioxane at room temperature for 1 hour.

According to step (d), the preparation of a compound of the formula (I) wherein either $R_1$ is the N terminus of any $\alpha$-amino acid or peptide sequence, or $R_2$ is the C terminus of any $\alpha$-amino acid or peptide sequence is carried out by liquid or solid-phase methodologies known to those skilled in the art: Bodanszky et al., "Peptide Synthesis", Interscience Publishers, 1966, or McOmie (ed.) "Protective Group in Organic Chemistry", Plenum Press, 1973, or Barany et al., "The Peptides: Analysis, Synthesis, Biology" 2, Chapter 1, Academic Press, 1980, or J. Stewart & J. Young, "Solid phase peptide synthesis", Pierce Chemical Company, 1984, or E. Atherthon & R. C. Sheppard, "Solid phase peptide synthesis: a practical approach", IRL Press, 1989, or S. A. Kates & F. Albericio (Eds.), "Solid-phase synthesis. A practical guide", Marcel Decker, 2000, or W. C. Chan & P. D. White (Eds.), "Fmoc solid phase peptide synthesis: a practical approach", Oxford University Press, 2000.

In the case of solid-phase synthesis any manual or automatic peptide synthesizer can be used and the peptides can be assembled in a stepwise manner on a resin support using either Boc or Fmoc strategies.

All the reagents used as starting materials are on the market or may be produced and purified in accordance with methods known in the art.

The deprotected peptides can be optionally purified by reverse phase high performance liquid chromatography as extensively described in the experimental part.

If desired, the salification of a compound of formula (I) with other acidic counterparts or the conversion of a corresponding salt thereof into the free compound (I) can be easily carried out according to well-known methods in the art.

As it will be appreciated by the person skilled in the art, when preparing the compounds of formula (I) object of the invention, optional functional groups within both the starting materials or the intermediates thereof and which could give rise to unwanted side reactions, need to be properly protected according to conventional techniques. Likewise, the conversion of these latter into the free deprotected compounds may be carried out according to known procedures.

It is also clear to the person skilled in the art that if a compound of formula (I), prepared according to the above process, is obtained as a mixture of isomers, their separation into the single isomers of formula (I), carried out according to conventional techniques, is still within the scope of the present invention.

Starting compounds of the formula (II) are known or can be obtained according to known methods.

For some references related to their preparation see: (a) de Macédo, P.; Marrano, C.; Keillor, J. W. Bioorg.

Med. Chem. 2002, 10, 355-360; (b) Sakura, N.; Itoh, T.; Uchida, Y.; Ohki, K.; Okimura, K.; Chiba, K.; Sato, Y.; Sawanishi, H. Bull. Chem. Soc. Japan 2004, 77, 1915-1924; (c) Varghese, J. PCT Int. Appl. 2003, 204, WO 2003045378; (d) Banwell, M. G.; McRae, K. J. J. Oil. Chem. 2001, 66, 6768-6774; (e) Boyle, W. J., Jr; Sifniades, S.; Van Peppen, J. F. J. Org. Chem. 1979, 44, 4841-4847.

For example, starting compounds of the formula (II) wherein $R_1$ is hydroxy, $R_2$ is a N-protecting group, preferably t-butoxycarbonyl or 9-fluorenylmethoxycarbonyl and n is as defined above are known or can be obtained according to known methods.

In particular, compounds of formula (II) wherein $R_1$ is hydroxy, $R_2$ is a N-protecting group, preferably t-butoxycarbonyl or 9-fluorenylmethoxycarbonyl and n is 1 or 2 were prepared starting from suitably protected asparagine or glutamine by Hofmann rearrangement using PIDA or PIFA in a mixture of DMF, water and pyridine, at a temperature ranging from 0 °C to room temperature and for a time varying from 15 to 24 hours. On the other hand, compounds of formula (II) wherein $R_1$ is hydroxy; $R_2$ is a

N-protecting group, preferably 9-fluorenylmethoxycarbonyl and n is 3 or 4 were prepared starting from $N_\alpha$-Fmoc-$N_\delta$-Boc-omithine or $N_\alpha$-Fmoc-$N_\varepsilon$-Boc-lysine by t-butoxycarbonyl- removal using trifluoroacetic acid in dichloromethane at a temperature ranging from 0 °C to room temperature and for a time varying from 5 to 24 hours or, alternatively, by using 4N hydrochloric in dioxane at a temperature ranging from 0 °C to room temperature and for a time varying from 5 to 48 hours.

According to a preferred embodiment, within the compounds of formula (II), $R_1$ is hydroxy, $R_2$ is 9-fluorenylmethoxycarbonyl.

Preferably, compounds of the formula (II) are prepared by reacting $N_\alpha$-Fmoc-asparagine or $N_\alpha$-Fmoc-glutamine with PIFA in a mixture of DMF, water and pyridine, at a temperature ranging from 0 °C to room temperature and for 24 hours, or by reacting $N_\alpha$-Fmoc-$N_\delta$-Boc-ornithine or $N_\alpha$-Fmoc-$N_\varepsilon$-Boc-lysine with trifluoroacetic acid in dichloromethane at room temperature for 5 hours.

The resultant starting compounds of the formula (II) can be either purified or used as crudes and further processed to afford the compounds of formula (I).

## Screening Methods

As stated above, the compounds of the formula (I) wherein either $R_1$ is the N terminus of any $\alpha$-amino acid or peptide sequence, or $R_2$ is the C terminus of any $\alpha$-amino acid or peptide sequence, or a salt thereof, can find applications in the FAXS "Fluorine chemical shift Anisotropy and eXchange for Screening" experiments for detection of inhibitors of protein-protein interactions. A peptide that represents the amino acid sequence of a protein responsible for the binding to another protein is tagged with one of the fluorinated aminoacids of the formula (I) according to the present invention.

FAXS is then applied to the protein-peptide complex for identification of molecules that displace the peptide from the protein.

The compounds of the formula (I) wherein either $R_1$ is the N terminus of any $\alpha$-amino acid or peptide sequence, or $R_2$ is the C terminus of any $\alpha$-amino acid or peptide sequence, or a salt thereof, can also find application in the 3-FABS "Three Fluorine

Atoms for Biochemical Screening" experiments for identification of inhibitors of an enzymatic reaction. A peptide that represents a substrate for an enzyme is tagged with one of the fluorinated amino acids of the formula (I) according to the present invention.

3-FABS is then applied to the enzyme-substrate solution for identification of molecules that inhibit the enzymatic reaction.

The compounds of the formula (I) wherein either $R_1$ is the N terminus of any $\alpha$-amino acid or peptide sequence, or $R_2$ is the C terminus of any $\alpha$-amino acid or peptide sequence, or a salt thereof can be used for the optimization of substrates for a given enzyme. Peptides tagged in different positions of the peptide aminoacid sequence with the fluorinated amino acids of the formula (I) according to the present invention are used with 3-FABS for identifying the most efficient substrate for the enzyme ($K_{cat}/K_M$).

In addition, the value of $K_{cat}$ and $K_M$ derived from the 3-FABS experiments provide structural information for identifying the moieties of the peptide responsible for the binding to the enzyme and those responsible for the catalytic reaction. This knowledge can then be used for generating a pharmacophore in the design of a potent inhibitor that competes with the substrate or of a transition state analogue.

The process involved in 3-FABS requires the following steps:

1. Determination of the linear range of the enzymatic reaction. This is achieved by monitoring the course of the reaction within the NMR tube. $^{19}F$ NMR spectra at different intervals are recorded and the $^{19}F$ signal integral of the substrate, that is a compound of the formula (I) as herein defined or of a product or products resulting from the cleavage, is plotted as a function of the time elapsed from the beginning of the reaction. Screening and KM measurements are then performed in an end point format.

The reaction is quenched with either a strong inhibitor, a denaturating, a chelating molecule or simply by lowering the pH of the solution after a delay for which the reaction is still linear and sufficient product is formed.

2. Determination of the $K_M$ of the **substrate** as defined above and cosubstrate. This is achieved by performing the reaction at different **substrate** or cosubstrate concentrations. The $K_M$ is determined using the Henry Michaelis Menten equation

$$V = \frac{[S]}{K_M + [S]} V_{max} \qquad (1)$$

where $V$ is the initial speed of the reaction and $V$max is the maximum speed ($V$max = Kcat*[$E_{tot}$], where $K_{cat}$ is the catalytic rate constant and [$E_{tot]}$ is the enzyme concentration) and [S] is the **substrate** concentration. V is obtained experimentally by measuring the integral of the $^{19}$F NMR signal of the product divided by the incubation time of the reaction. A plot of these values as a function of [S] permits the determination of $K_M$ and $V$max.

3. Screening is performed in an end point format using either chemical mixtures or single compound at a **substrate** concentration in the range 2-5 $K_M$ (balanced assay condition). In every screening run a sample without test molecules is recorded. This sample represents the reference with 0% inhibition. Deconvolution of the active mixtures is then performed for the identification of the inhibitors.

4. The $IC_{50}$ value of the detected inhibitor is obtained by recording experiments at different inhibitor concentration and by monitoring the integral of the product or **substrate** $^{19}$F NMR signal. A plot of these values as a function of the inhibitor concentration allows the determination of $IC_{50}$ according to the equations:

a) monitoring the substrate signal

$$[S_w] = \frac{[S_{w/o}] - [S_{TOT}]}{1 + \left(\frac{[I]}{IC_{50}}\right)^n} + [S_{TOT}] \qquad (2)$$

b) monitoring the product signal

$$[P_w] = \frac{[P_{w/o}]}{1 + \left(\frac{[I]}{IC_{50}}\right)^n} \qquad (3)$$

where [Sw] and [*Sw*/*o*] are given by the integrals of the **substrate** signal in the presence and absence of the inhibitor, respectively, [*Pw*] and [*Pw*/*o*] are given by the integrals of the product or products signal in the presence and absence of the inhibitor, respectively, and [$S_{TOT}$] = [*Pw*/*o*] + [*Sw*/*o*] = [*Pw*] + [*Sw*]. [$S_{TOT}$] is the **substrate** concentration used for the experiments and it represents an internal reference, [*I*] is the concentration of the inhibitor, $IC_{50}$ the concentration of the inhibitor at which 50% inhibition is observed and n is the Hill coefficient.

With the aim to better illustrate the present invention, without posing any limitation to it, the following examples are now given.

## General Preparation Methods

Flash chromatography was performed on silica gel (Merck grade 9385, 60Å).

HPLC/MS detailed descriptions of both the analytical and the purification methods were reported in the experimental part devoted to peptides synthesis.

$^{1}$H-NMR spectroscopy was performed on a Mercury VX 400 operating at 400.45 MHz equipped with a 5mm double resonance probe (1H {15N-31P} ID_PFG Varian) or on a INOVA 500 operating at 499.76 MHz.

$^{19}$F-NMR spectroscopy was performed at 20 °C on a Varian Inova 600 MHz NMR spectrometer operating at

a $^{19}F$ Larmor frequency of 564 MHz. A 5-mm probe with inner and outer coils tunable to either $^{19}F$ or $^{1}H$ frequency was used.

**Glossary**

In this .specification, the abbreviations used for α-amino acids and protecting groups are based on recommendations of the IUPAC-IUB Commission on Biochemical Nomenclature (see Eur. J. Biochem. 1984, 138, 9-37). Furthermore, the following abbreviations were used throughout the text: Boc, tert-butyloxycarbonyl. DCM, dichloromethane. DIEA, diisopropylethylamine. DMA, N,N-dimethylacetamide. DMF, N,N-dimethylformamide. DMSO, dimethyl sulfoxide. DTT, dithiothreitol. Fmoc, 9-fluorenylmethoxycarbonyl. NMP, N-methyl-2-pyrrolidinone. PBF, 2,2,4,6,7-pentamethyldihydrobenzofuran-5-sulfonyl. PIDA, iodosobenzene diacetate. PIFA, [bis(trifluoroacctoxy)iodo]benzene. PyBOP®, benzotriazole-1-yl-oxy-tris(pyrrolidino)-phosphonium hexafluorophosphate. tBu, tert-butyl. TFA, trifluoroacetic acid. TIS, triisopropylsilane. TRIS, tris(hydroxymethyl)aminomethane.
According to the present invention the α-amino acids could be either in the L or D configuration.
PyBOP®, Rink Amide AM resin and amino acid derivatives were purchased from Novabiochem, Switzerland. Fmoc-Ile-Rink Amide MBHA resin was purchased from Polymer Laboratories.
The compounds **1-20** were prepared according to the following procedures:

**Preparation A**

**Procedure for the Hofmann rearrangement (Scheme 2)**

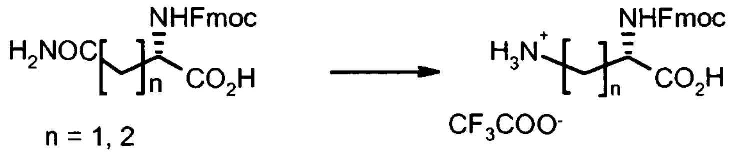

n = 1: **1**
n = 2: **2**

Scheme 2

**(S)-3-Amino-2-(9H-fluoren-9-ylmethoxycarbonylamino)-propionic acid trifluoroacetate (1)** [Compound of formula (II) wherein $R_1$ is hydroxy, $R_2$ is 9-fluorenylmethoxycarbonyl and n is 1]

To a stirred suspension of $N_\alpha$-Fmoc-asparagine (2 g, 5.65 mmol) in a 2:1 mixture of DMF and water (51 mL), cooled at 0 °C, PIFA (4.24 g, 9.86 mmol) was added. After 15 minutes pyridine (1.08 mL, 2.32 mmol) was added dropwise at 0 °C, and the reaction mixture was allowed to reach room temperature. The suspension became a clear yellowish solution after three to four hours, and was left under stirring for 24 hours. Evaporation of the solvents afforded a deep red oil, that was washed with n-hexane (3 x 30 mL) and ether (3 x 30 mL). The reaction crude was then crystallized from water (25 mL). The light-yellow solid was then filtered, washed with cold water and dried, delivering compound 1 (1.83 g, 73% yield).
$^{1}H$ NMR (400 MHz, DMSO-*d6*) δ ppm 7.92 (d, *J*=7.56 Hz, 2 H) 7.72 (d, *J*=8.14 Hz, 2 H) 7.50 (d, *J*=7.22 Hz, 1H) 7.44 (t, *J*=6.88 Hz, 2 H) 7.35 (d, *J*=1.10 Hz, 2 H) 4.22 - 4.44 (m, 3 H) 4.08 - 4.21 (m, 1 H) 3.09 - 3.19 (m, 1 H) 2.98 - 3.08 (m, 1 H) HRMS (ES+): m/z: calcd for $C_{18}H_{19}N_2O_4$ : 327.1339 $[M+H]^+$; found: 327.1349

**(S)-4-Amino-2-(9H-fluoren-9-ylmethoxycarbonylamino)-butyric acid trifluoroacetate (2)** [Compound of formula (II) wherein $R_1$ is hydroxy, $R_2$ is 9-fluorenylmethoxycarbonyl and n is 2]

Using the same aforementioned protocol, starting from $N_\alpha$-Fmoc-glutamine (4.12 g, 11.2 mmol), compound 2 was obtained. Due to the higher solubility in water compared to 1, it was used after washing with n-hexane and ether without any further purification (4.5 g, estimated 71% yield based on HPLC purity of 85% at 220 nm).
$^{1}H$ NMR (400 MHz, DMSO-*d6*) δ ppm 7.90 (d, *J*=7.56 Hz, 2 H) 7.63 - 7.79 (m, 6 H) 7.43 (t, *J*=7.32 Hz, 2 H) 7.34 (t,

*J*=7.44 Hz, 2 H) 4.19 - 4.42 (m, 3 H) 4.04 - 4.16 (m, I H) 2.77 - 2.93 (m, 2 H) 1.96 - 2.07 (m, 1 H) 1.82 1.96 (m, 1H) HRMS (ES+): m/z: calcd for $C_{19}H_{21}N_2O_4$: 341.1496 [M + H]$^+$; found: 341.1496

**Preparation B**

**Procedure for the t-butoxycarbonyl-protective group removal (Scheme 3)**

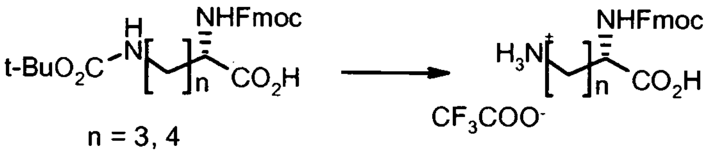

n = 3: **3**
n = 4: **4**

Scheme 3

**(S)-5-Amino-2-(9H-fluoren-9-ylmethoxycarbonylamino)-pentanoic acid trifluoroacetate (3)** [Compound of formula (II) wherein $R_1$ is hydroxy, $R_2$ is 9-fluorenylmethoxycarbonyl and n is 3]

To a stirred solution of $N_\alpha$-Fmoc-$N_\delta$-Boc-ornithine (1 g, 2.2 mmol) in dichloromethane (5 mL) trifluoroacetic acid (5 mL) was added, and the reaction mixture was stirred at room temperature for 5 hours. The light-yellow solution was then evaporated to dryness, affording compound 3 (white foam) (1.01 g, 98% yield) that was used in the next step without any further purification.
$^1$H NMR (400 MHz, DMSO-*d*$_6$) δ ppm 7.90 (d, *J*=7.44 Hz, 2 H) 7.72 (d, *J*=8.17 Hz, 2 H) 7.58-7.69 (m,4H) 7.43 (t, *J*=7.19Hz, 2H) 7.33 (t, *J*=7.99Hz, 2H) 4.17-4.41 (m, 3 H) 3.89 - 4.05 (m, 1 H) 2.71 - 2.87 (m, 2 H) 1.72 - 1.87 (m, 1 H) 1.53 - 1.69 (m, 3 H) HRMS (ES+): m/z: calcd for $C_{20}H_{23}N_2O_4$ : 355.1652 [M + H]$^+$; found: 355.1656

**(S)-6-Amino-2-(9H-fluoren-9-ylmethoxycarbonylamino)-hexanoic acid trifluoroacetate (4)** [Compound of formula (II) wherein $R_1$ is hydroxy, $R_2$ is 9-fluorenylmethoxycarbonyl and n is 4]

Using the same aforementioned protocol, starting from or $N_\alpha$-Fmoc-$N_\varepsilon$-Boc-lysine (1 g, 2.13 mmol), compound **4** was obtained (1.05 g, quantitative yield) as a white foam.
$^1$H NMR (400 MHz, DMSO-*d*$_6$) δ ppm 7.92 (d, *J*=7.56 Hz, 2 H) 7.69 - 7.80 (m, 2 H) 7.57 - 7.66 (m, 5 H) 7.43 (t, *J*=7.96 Hz, 2 H) 7.36 (t, *J*=6.87 Hz, 2 H) 4.18 - 4.42 (m, 3 H) 3.85 - 4.05 (m, I H) 2.72 - 2.89 (m, 2 H) 1.68 - 1.80 (m, 1 H) 1.46 - 1.68 (m, 3 H) 1.32 - 1-.46 (m, 2 H)
HRMS (ES+): m/z: calcd for $C_{21}H_{25}N_2O_4$ 369.1809 [M + H]$^+$; found: 369.1811

**Procedure for the double reductive amination reaction (Scheme 4)**

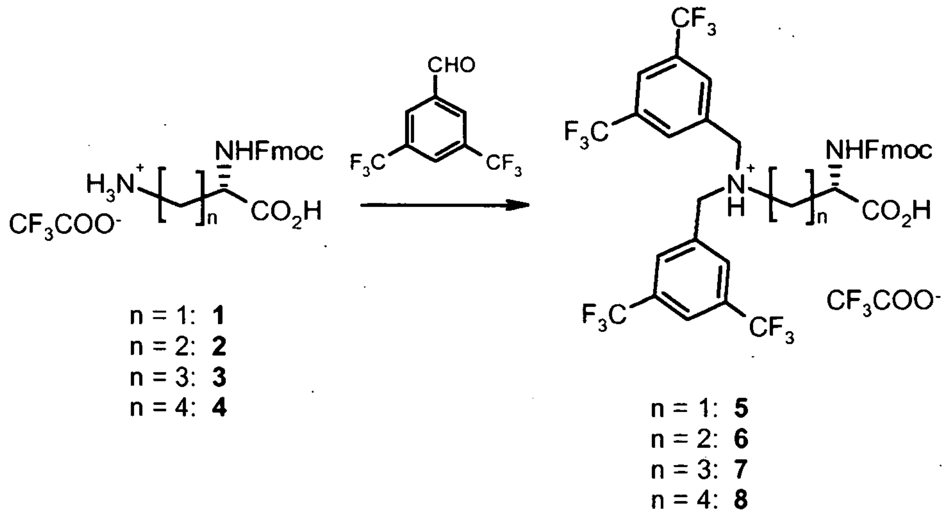

Scheme 4

Example 1

**(S)-3-[Bis-(3,5-bis-trifluoromethyl-benzyl)-amino]-2-(9H-fluoren-9-ylmethoxycarbonylamino)-propionic acid trifluoroacetate, (5)** [Compound of formula (I) wherein R is 3,5-bis(trifluoromethyl)benzyl, $R_1$ is hydroxy, $R_2$ is 9-fluorenylmethoxycarbonyl and n is 1]

To a solution of 1 (120 mg, 0.27 mmol) in DMF (5 mL), stirred at room temperature, sodium triacetoxyborohydride (506 mg, 2.4 mmol) and 3,5-bis(trifluoromethyl)benzaldehyde (0.26 mL, 1.59 mmol) were subsequently added.
After 5 minutes, trifluoroacetic acid (0.5 mL) was added, and the reaction mixture was stirred at room temperature for 3.5 hours. The reaction mixture was then evaporated to dryness *in vacuo,* taken up in dichloromethane and the organic phase was washed with water and brine, dried over $Na_2SO_4$ and concentrated. The crude was then purified by flash chromatography (Cyclohexane/AcOEt: 50/50, AcOEt and then AcOEt/MeOH: 80/20) to afford compound 5 as a light-yellow solid (107 mg, 45% yield).
$^1$H NMR (400 MHz, DMSO-$d_6$) δ ppm 7.86 - 7.93 (m, 6 H) 7.75 (s, 2 H) 7.68 (d, *J*=7.56 Hz, 2 H) 7.37 - 7.46 (m, 2 H) 7.26 - 7.35 (m, 2 H) 7.03 (br s, 1 H) 4.07 - 4.33 (m, 4 H) 3.77 - 3.97 (m, 4 H) 3.09 (d, *J*=7.89 Hz, 1 H) 2.91 (d, *J*=7.89 Hz, 1 H) HRMS (ES+): m/z: calcd for $C_{36}H_{27}F_{12}N_2O_4$: 779.1774 [M + H]$^+$; found: 779.1797 $^{19}$F NMR (DMSO-$d_6$): 11.88 ppm.

Example 2

**(S)-4-[Bis-(3,5-bis-trifluoromethyl-benzyl)-amino]-2-(9H-fluoren-9-**ylmethoxycarbonylamino)-butyric acid trifluoroacetate (6) [Compound of formula (I) wherein R is 3,5-bis(trifluoromethyl)benzyl, $R_1$ is hydroxy, $R_2$ is 9-fluorenylmethoxycarbonyl and n is 2]

Using the same protocol described for compound 5, starting from 2 (96 mg of crude, corresponding to 82 mg of pure 2, 0.18 mmol), compound 6 was obtained (100 mg, 61% yield) as a white solid.
m.p. 152 °C, $[\alpha]_D$ +1.2 (c 1, MeOH)
$^1$H NMR (400 MHz, DMSO-$d_6$) δ ppm 7.73 - 7.92 (m, 8 H) 7.65 (d, *J*=7.93 Hz, 2 H) 7.25 - 7.46 (m, 4 H) 6.91 (s, 1 H) 4.12 - 4.32 (m, 3 H) 3.69 - 3.95 (m, 5 H) 2.73 (t, *J*=6.58 Hz, 2 H) 1.82 - 2.14 (m, 2 H)
HRMS (ES+): m/z: calcd for $C_{37}H_{29}F_{12}N_2O_4$: 793.1930 [M + H]$^+$; found: 793.1957 $^{19}$F NMR (DMSO-$d_6$): 11.84 ppm

Example 3

**(S)-5-[Bis-(3,5-bis-trifluoromethyl-benzyl)-amino]-2-(9H-fluoren-9-ylmethoxycarbonylamino)-pentanoic acid trifluoroacetate (7)** [Compound of formula (I) wherein R is 3,5-bis(trifluoromethyl)benzyl, $R_1$ is hydroxy, $R_2$ is 9-fluorenylmethoxycarbonyl and n is 3]

Using the same protocol described for compound **5**, starting from **3** (125 mg, 0.27 mmol), compound 7 was

obtained (151 mg, 61% yield) as a white solid.
m.p. 141 °C, $[\alpha]_D$ +3.0 (c 1, MeOH)
$^1$H NMR (400 MHz, DMSO-$d_6$) δ ppm 7.77 - 7.93 (m, 8 H) 7.65 (d, *J*=7.68 Hz, 2 H) 7.35 - 7.42 (m, 2 H) 7.24 - 7.33 (m, 2 H) 6.77 - 7.08 (br s, 1 H) 4.14 - 4.34 (m, 3 H) 3.70 - 3.87 (m, 5 H) 2.62 (t, *J*=6.43 Hz, 2 H) 1.73 - 1.86 (m, 1H) 1.47 - 1.72 (m, 3 H) HRMS (ES+): m/z: calcd for $C_{38}H_{31}F_{12}N_2O_4$ : 807.2087 $[M + H]^+$; found: 807.2093 $^{19}$F NMR (DMSO-$d_6$): 11.83 ppm

**Example 4**

**(S)-6-[Bis-(3,5-bis-trifluoromethyl-benzyl)-amino]-2-(9H-fluoren-9-ylmethoxycarbonylamino)-hexanoic acid trifluoroacetate (8)** [Compound of formula (1) wherein R is 3,5-bis(trifluoromethyl)benzyl, $R_1$ is hydroxy, $R_2$ is 9-fluorenylmethoxycarbonyl and n is 4]

Using the same same protocol described for compound **5**, starting from **4** (128 mg, 0.27 mmol), compound **8** was obtained (152 mg, 60% yield) as a white solid.
m.p. 121 °C, $[\alpha]_D$ -2.3 (c 1, MeOH)
$^1$H NMR (400 MHz, DMSO-$d_6$) δ ppm 7.78 - 7.9 (m, 8 H) 7.62 (d, *J*=7.72 Hz, 2 H) 7.32 - 7.38 (t, 2 H) 7.22 - 7.3 (t, *J*=6.22 Hz, 2 H) 6.62 - 6.72 (br s, 1 H) 4.15 - 4.23 (m, 3 H) 3.65 - 3.78 (m, 5 H) 2.58 (t, *J*=6.54 Hz, 2 H) 1.62 - 1.78 (m, 1 H) 1.43 - 1.58 (m, 3 H) 1.19-1.3(m,2H)
HRMS (ES+): m/z: calcd for $C_{39}H_{33}F_{12}N_2O_4$ : 821.2243 $[M + H]^+$; found: 821.2273 $^{19}$F NMR (DMSO-$d_6$): 11.83 ppm

**Example 5**

**Procedure for the conversion of compounds 5 through 8 into their corresponding hydrochloride salts**

Pure TFA salts (5 through 8) were dissolved in dry dioxane (1 mmol in 16 mL) and exposed to 4N solution of hydrochloric acid in dioxane (4 mL). The resulting solutions were stirred at room temperature for 1 hour and then evaporated to dryness. The corresponding hydrochloride salts were subsequently used without any further purification.

**Procedure for the reductive amination reaction (Scheme 5)**

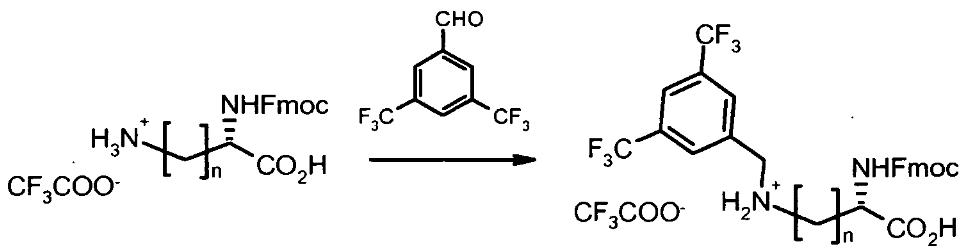

n = 2: **2**
n = 3: **3**
n = 4, **4**

n = 2: **9**
n = 3: **10**
n = 4, **11**

Scheme 5

**Example 6**

**(S)-4-(3,5-Bis-trifluoromethyl-benzylamino)-2-(9H-fluoren-9-ylmethoxycarbonylamino)-butyric acid trifluoroacetate (9)** [Compound of formula (1) wherein R is hydrogen, $R_1$ is hydroxy, $R_2$ is 9-fluorenylmethoxycarbonyl and n is 2]

To a solution of 2 (208 mg of crude, corresponding to 177 mg of pure 2, 0.39 mmol) in DMF (5 mL), stirred at room temperature, sodium triacetoxyborohydride (290 mg, 1.4 mmol) and 3,5-bis(trifluoromethyl)benzaldehyde (0.07 mL, 0.43 mmol) were subsequently added. After 20 minutes, trifluoroacetic acid (0.5 mL) was added, and the reaction mixture was stirred at room temperature for 1 hour. The reaction mixture was then poured into water (20 mL) and the pH was adjusted to 6-7, using aqueous NaOH 32 w/w. The aqueous phase was then extracted with AcOEt (2 X 20 mL). The combined organic phases were washed with water (3 X 50 mL) and brine, dried over $Na_2SO_4$ and evaporated to dryness. The crude was finally purified by flash chromatography (dichloromethane, dichloromethane/MeOH: 95/5, dichloromethane/MeOH: 80/20) to afford compound **9** as a white solid (114 mg, 43% yield).
m.p.> 160 °C (decomposition), $[\alpha]_D$ +1.1 (*c* 1, MeOH)
1H NMR (400 MHz, DMSO-$d_6$) δ ppm 8.09 (s, 2 H) 7.99 (s, 1H) 7.89 (d, *J*=7.44 Hz, 2 H) 7.69 (d, *J*=7.93 Hz, 2 H) 7.42 (t, *J*=7.32 Hz, 2 H) 7.24 - 7.37 (m, 3 H) 4.13 - 4.37 (m, 3 H) 3.88 - 4.08 (m, 3 H) 2.61 - 2.79 (m, 2 H) 1.85 - 2.00 (m, 1 H) 1.72 - 1.85 (m, 1 H)
HRMS (ES+): m/z: calcd for $C_{28}H_{25}F_6N_2O_4$: 567.1713 $[M + H]^+$; found: 567.1693
$^{19}F$ NMR (DMSO-$d_6$) mixture of rotational isomers: 12.15 ppm (major), 12.02 ppm (minor);
$^{19}F$ NMR (50 mM Tris, 1 mM DTT, 0.001% Triton® X-100, pH = 7.5) mixture of rotational isomers: 12.83 ppm (major), 12.81 ppm (minor)

**Example 7**

**(S)-5-(3,5-Bis-trifluoromethyl-benzylamino)-2-(9H-fluoren-9-ylmethoxycarbonylamino)-pentanoic acid trifluoroacetate (10)** [Compound of formula (I) wherein R is hydrogen, $R_1$ is hydroxy, $R_2$ is 9-fluorenylmethoxycarbonyl and n is 3]

Using the same protocol described for compound **9**, starting from 3 (2.2 g, 4.7 mmol), compound **10** was obtained (1.8 g, 55% yield) as a white solid.
m.p. 162 °C, $[\alpha]_D$ +7.3 (c 1, MeOH)
$^1H$ NMR (400 MHz, DMSO-$d_6$) δ ppm 8.06 (s, 2 H) 7.96 (s, 1 H) 7.89 (d, *J*=7.44 Hz, 2 H) 7.71 (d, *J*=7.44 Hz, 2 H) 7.42 (t, *J*=7.44 Hz, 2 H) 7.27 - 7.37 (m, 3 H) 4.19 - 4.33 (m, 3 H) 3.84 - 3.94 (m, 3 H) 2.52 - 2.58 (m, 2 H) 1.73 - 1.83 (m, I H) 1.59 - 1.71 (m, 1 H) 1.48 - 1.59 (m, 2 H)
HRMS (ES+): m/z: calcd for $C_{29}H_{27}F_6N_2O_4$: 581.1870 $[M + H]^+$; found: 581.1875
$^{19}F$ NMR (50 mM Tris, 1 mM DTT, 0.001% Triton® X-100, pH = 7.5): 12.78 ppm

**Example 8**

**(S)-6-(3,5-Bis-trifluoromethyl-benzylamino)-2-(9H-fluoren-9-ylmethoxycarbonylamino)-hexanoic acid trifluoroacetate (11)** [Compound of formula (I) wherein R is hydrogen, $R_1$ is hydroxy, $R_2$ is 9-fluorenylmethoxycarbonyl and n is 4]

Using the same protocol described for compound 9, starting from 4 (2.8 g, 5.81 mmol), compound 11 was obtained (2.23 g, 54% yield) as a white solid.
m.p.> 160 °C (decomposition)
$^1H$ NMR (400 MHz, DMSO-$d_6$) δ ppm 8.04 (s, 2 H) 7.94 (s, 1 H) 7.89 (d, *J*=7.44 Hz, 2 H) 7.69 (d, *J*=6.95 Hz, 2 H) 7.43 (t, *J*=7.44 Hz, 2 H) 7.28 - 7.38 (m, 3 H) 4.17 - 4.33 (m, 3 H) 3.73 3.93 (m, 3 H) 2.43 - 2.52 (m, 2 H) 1.65 - 1.79 (m, 1 H) 1.51 - 1.63 (m, 1H) 1.28-1.49(m,4H)
HRMS (ES+): m/z: calcd for $C_{30}H_{29}F_6N_2O_4$: 595.2026 $[M + H]^+$; found: 595.2047
$^{19}F$ NMR (50 mM Tris, 1 mM DTT, 0.001% Triton®X-100, pH = 7.5): 12.76 ppm

**Procedure for the introduction of the *t*-butoxycarbonyl- group (boc, Scheme 6)**

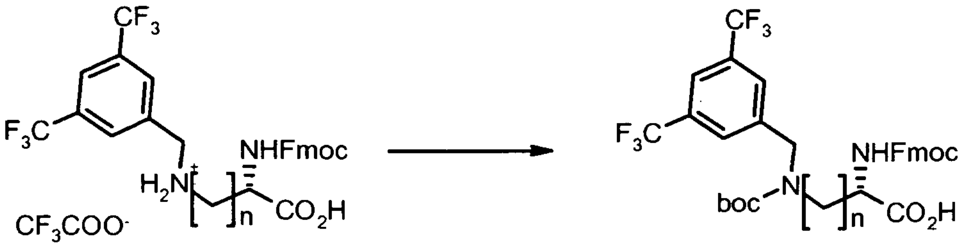

n = 2: **9**
n = 3: **10**
n = 4: **11**

n = 2: **12**
n = 3: **13**
n = 4: **14**

Scheme 6

**Example 9**

**(S)-4-[(3,5-Bis-trifluoromethyl-benzyl)-tert-butoxycarbonyl-amino]-2-(9H-fluoren-9-ylmethoxycarbonylamino)-butyric acid (12)** [Compound of formula (I) wherein R is teit-butoxycarbonyl, $R_1$ is hydroxy, $R_2$ is 9-fluorenylmethoxycarbonyl and n is 2]

To a stirred solution of compound 9 (200 mg, 0.29 mmol) in AcOEt (2 mL), saturated aqueous $NaHCO_3$ (2 mL) and di-tert-butyl dicarbonate (256 mg, 1.18 mmol) were added. The biphasic system was swirled for 3 hours at room temperature. The reaction mixture was diluted with additional dichloromethane; the organic phase was separated, washed with water and then with brine. Finally, it was dried over $Na_2SO_4$ and evaporated to dryness. The crude was purified by flash chromatography (dichloromethane, dichloromethane/MeOH: 95/5, dichloromethane/MeOH: 90/10) to afford compound 12 (160 mg, 83% yield) as a white foam.
m.p. 178 °C, $[\alpha]_D$ +5.8 (c 1, MeOH)
1H NMR (400 MHz, DMSO-$d_6$) $\delta$ ppm 8.01 (s, 1 H) 7.81 - 7.94 (m, 4 H) 7.68 (d, *J*=7.80 Hz, 2 H) 7.41 (t, *J*=7.44 Hz, 2 H) 7.23 - 7.35 (m, 3 H) 7.01 (br s, 1 H) 4.54 (br s, 2 H) 4.17 - 4.33 (m, 3 H) 3.74 (br s, 1H) 3.32 - 3.45 (m, 2 H) 1.91 - 2.05 (m, 1H) 1.73 - 1.85 (m, 1H) 1.28 - 1.45 (br s, 9 H)
HRMS (ES+): m/z: calcd for $C_{33}H_{33}F_6N_2O_6$: 667.2237 $[M + H]^+$; found: 667.2266
$^{19}F$ NMR (50 mM Tris, 1 mM DTT, 0.001% Triton® X-100, pH = 7.5) mixture of rotational isomers: 12.88 ppm (major), 12.83 ppm (minor)

**Example 10**

**(S)-5-[(3,5-Bis-trifluoromethyl-benzyl)-tert-butoxycarbonyl-amino]-2-(9H-fluoren-9-ylmethoxycarbonylamino)-pentanoic acid (13)** [Compound of formula **(I)** wherein R is *tert*-butoxycarbonyl, $R_1$ is hydroxy, $R_2$ is 9-fluorenylmethoxycarbonyl and n is 3]

Using the same protocol described for compound **12**, starting from **10** (195 mg, 0.28 mmol), and using dichloromethane (2 mL) instead of AcOEt, compound 13 was obtained (195 mg, 98% yield) as a white foam.
m.p.> 166 °C (decomposition), $[\alpha]_D$ +5.0 (c 1, MeOH)
1H NMR (400 MHz, DMSO-$d_6$) $\delta$ ppm 8.00 (s, 1 H) 7.84 - 7.94 (m, 4 H) 7.69 (d, *J*=7.56 Hz, 2 H) 7.42 (t, *J*=7.28 Hz, 2 H) 7.27 - 7.37 (m, 3 H) 7.06 (br s, 1 H) 4.51 (br s, 2 H) 4.16 - 4.32 (m, 3 H) 3.78 (br s, 1 H) 3.17 - 3.33 (m, 2 H) 1.65 - 1.75 (m, I H) 1.48 - 1.62 (m, 3 H) 1.19 - 1.48 (br s, 9 H)
HRMS (ES+): m/z: calcd for $C_{34}H_{35}F_6N_2O_6$: 681.2394 $[M + H]^+$; found: 681.2425
$^{19}F$ NMR (50 mM Tris, 1 mM DTT, 0.001% Triton® X-100, pH = 7.5): 12.83 ppm

## Example 11

**(S)-6-[(3,5-Bis-trifluoromethyl-benzyl)-tert-butoxycarbonyl-amino]-2-(9H-fluoren-9-ylmethoxycarbonylamino)-hexanoic acid (14)** [Compound of formula (I) wherein R is *tert*-butoxycarbonyl, $R_1$ is hydroxy, $R_2$ is 9-fluorenylmethoxycarbonyl and n is 4]

Using the same protocol described for compound **12,** starting from **11** (200 mg, 0.29 mmol), and using dichloromethane (2 mL) instead of AcOEt, compound **14** was obtained (185 mg, 98% yield) as a white foam.
m.p. 68 °C, $[\alpha]_D$ -4.1 (*c* 1, MeOH)
1H NMR (400 MHz, DMSO-$d_6$) δ ppm 8.02 (s, 1 H) 7.84 - 7.95 (m, 4 H) 7.72 (d, *J*=7.44 Hz, 2 H) 7.59 (d, *J*=8.41 Hz, 1 H) 7.42 (t, *J*=7.44 Hz, 2 H) 7.33 (d, *J*=7.56 Hz, 2 H) 7.10(brs, 1 H) 4.48 - 4.60 (m, 2 H) 4.17 - 4.36 (m, 3 H) 3.85 - 3.98 (m, 1 H) 3.14 - 3.28 (m, 2 H) 1.22 - 1.75 (m, 15 H)
HRMS (ES+): m/z: calcd for $C_{35}H_{37}F_6N_2O_6$: 695.2550 $[M + H]^+$; found: 695.2574
$^{19}F$ NMR (50 mM Tris, 1 mM DTT, 0.001% Triton® X-100, pH = 7.5): 12.84 ppm

## Example 12

**Procedure for the solid phase synthesis of peptides 15-20, compounds of the formula (Ib)**

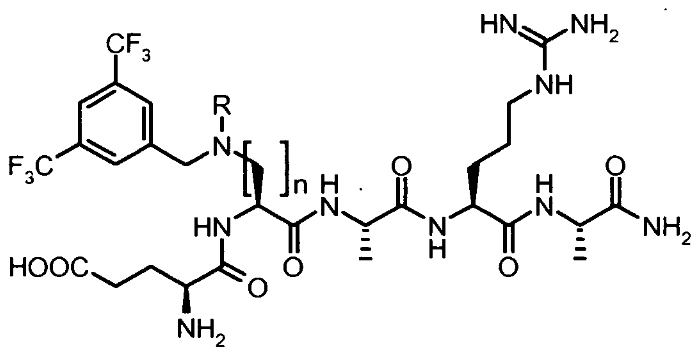

**(Ib)**

The syntheses of peptides **15-20** have been achieved on solid phase starting from the commercially available Rink Amide AM resin, 4-(2',4'-dimethoxyphenyl-Fmoc-aminomethyl)-phenoxyacetamido-norleucyl-aminomethyl copoly (styrene-1%DVB).

The Fmoc-deprotection step was performed on the Rink apparatus with rapid (1 minute) and multiple (usually 14-15) treatments with the cleavage mixture piperidine/DMA 20%. Double coupling step was performed (60 and 120 min, respectively), using each time 2 equiv. of the appropriate amino acid, 2 equiv. of PyBOP® and 10-12 equiv. of DIEA (Hunig's base), in DMA as solvent. A capping step usually followed: two 5 minutes reactions with the mixture DMA/$Ac_2O$/DIEA 80:15:5. The resin was always pre-swelled with DCM and DMA and accurately washed with DMA, NMP and 2-propanol after coupling, capping and de-protection steps.

Finally, the peptides were cleaved from the resin as C-terminal amides by treatment with a mixture of TFA/TIS/$H_2O$ 95/2.5/2.5, repeated twice for 60 and 30 minutes, respectively. The crude pentapeptides **(15-20)** were submitted to HPLC purification as described hereafter.

Peptides **15-17** were analyzed by HPLC/MS using Waters Alliance HT 2795 Separation module™ LC, equipped with UV-VIS detector Waters 996 PDA and MS detector Waters ZQ™ (single quadrupole), equipped with an electrospray (ESI +/-) ion source. Column was Waters XTerra RP18, 5 μm, 4.6X250 mm. Mobile phase A was 0.1% trifluoroacetic acid/acetonitrile 95/5. Mobile phase B was acetonitrile/water 95/5. HPLC Retention Times are given in minutes at 220 nm or 254 nm, according to the following LC conditions: gradient from 10% to 90% B in 15 minutes, hold 90% B for 1 minute. Flow rate 1 mL/min. Injection volume 10 μL. Column temperature 30 °C. Mass are given as *m*/*z* ratio. Full scan, mass range from 100 to 1500 amu. Capillary voltage was 3.48 kV (ES+) and 2.76 kV (ES-). Source temperature was 120 °C. Cone Voltage was 15 V (ES+) and 27 V (ES-).

Peptides **15-17** were purified by preparative HPLC/MS by using Waters FractionLynx System LC, equipped with UV-VIS detector Waters 2996 PDA and MS detector Waters ZQ™ (single quadrupole), equipped with an electrospray (ESI +/-) ion source. Column was Waters XTerra Prep RP18, 10 μm, 19X250 mm. Mobile phase A was 0.1% trifluoroacetic acid/acetonitrile 95/5. Mobile phase B was acetonitrile. LC conditions: gradient from 10% to 90% B in 15 minutes, hold

90% B for 1 minute. Flow rate 20 mL/min. Column temperature ambient. Mass are given as *m*/*z* ratio. Full scan, mass range from 100 to 1500 amu. Capillary voltage was 3.25 kV (ES+) and 2.75 kV (ES-). Source temperature was 120 °C. Cone Voltage was 18 V (ES+) and 18 V (ES-).

Peptides **18-20** were analyzed by HPLC/MS by using Waters Acquity UPLC™ System LC, equipped with UV-VIS detector Waters 2996 PDA and MS detector Waters ZQ™ (single quadrupole), equipped with an electrospray (ESI +/-) ion source. Column was Waters Acquity UPLC™ BEH C18, 1.7 μm, 2.1X50 mm. Mobile phase A was 0.05% $NH_3$ (pH 10)/acetonitrile 95/5. Mobile phase B was acetonitrile/water 95/5. HPLC Retention Times are given in minutes at 220 nm or 254 nm, according to the following LC conditions: gradient from 5% to 95% B in 2 minutes. Flow rate 0.8 mL/min. Injection volume 2 μL. Column temperature 45 °C. Mass are given as *m*/*z* ratio. Full scan, mass range from 100 to 1200 amu. Capillary voltage was 3.5 kV (ES+) and 2.8 kV (ES-). Source temperature was 120°C. Cone Voltage was 14 V (ES+) and 28 V (ES-).

Peptides **18-20** were purified by preparative HPLC/MS by using Waters FractionLynx System LC, equipped with UV-VIS detector Waters 2996 PDA and MS detector Waters ZQ™ (single quadrupole), equipped with an electrospray (ESI +/-) ion source. Column was Waters XTerra Prep RP18, 5 μm, 19X100 mm. Mobile phase A was 0.05% $NH_3$ (pH 10)/acetonitrile 95/5. Mobile phase B was acetonitrile. LC conditions: gradient from 0% to 70% B in 8 minutes, then to 100% B in 1 minute, hold 100% B for 2 minutes. Flow rate 20 mL/min. Column temperature ambient. Mass are given as *m*/*z* ratio. Full scan, mass range from 100 to 1200 amu. Capillary voltage was 3.25 kV (ES+) and 2.75 kV (ES-). Source temperature was 120 °C. Cone Voltage was 18 V (ES+) and 18 V (ES-).

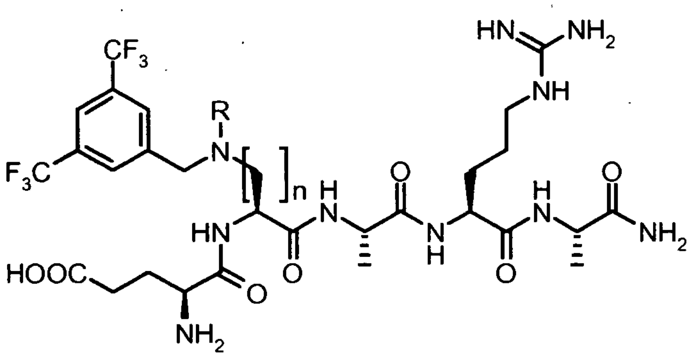

**(Ib)**

**E $X_1$ A R A-$NH_2$ trifluoroacetate (15)** [SEQ ID NO.:1]

In this compound $X_1$ is the fluorinated amino acid of formula (Ib) wherein R is 3,5-bis(trifluoromethyl)benzyl and n is 2.

Retention time: 9.87 min; MW (monoisotopic) calcd for $C_{39}H_{48}F_{12}N_{10}O_7$: 996.35 MS (ES+): 998.34 $[M + H]^+$, 500.02 $[M + 2H]^{+2}/2$; MS (ES-): 996.37 $[M - H]^{-19}$$^{19}F$ NMR (50 mM Tris, 1 mM DTT, 0.001% Triton® X-100, pH = 7.5): 12.81 ppm

**E $X_2$ A R A-$NH_2$ trifluoroacetate (16)** [SEQ ID NO.:2]

In this compound $X_2$ is the fluorinated amino acid of formula (Ib) wherein R is 3,5-bis(trifluoromethyl)benzyl and n is 3.

Retention time: 9.47 min; MW (monoisotopic) calcd for $C_{40}H_{50}F_{12}N_{10}O_7$: 1010.37
MS (ES+): 1012.36 $[M + H]^+$, 507.00 $[M + 2H]^{+2}/2$
$^{19}F$ NMR (50 mM Tris, 1 mM DTT, 0.001% Triton® X-100, pH = 7.5): 12.80 ppm

**E $X_3$ A R A-$NH_2$ trifluoroacetate (17)** [SEQ ID NO.:3]

In this compound $X_3$ is the fluorinated amino acid of formula (Ib) wherein R is 3,5-bis(trifluoromethyl)benzyl and n is 4.

Retention time: 9.50 min; MW (monoisotopic) calcd for $C_{41}H_{52}F_{12}N_{10}O_7$: 1024.38
MS (ES+): 1026.45 $[M + H]^+$, 514.06 $[M + 2H]^{+2}/2$; MS (ES-): 1024.46 $[M - H]^-$
$^{19}F$ NMR (50 mM Tris, 1 mM DTT, 0.00 1 % Triton® X-100, pH = 7.5): 12.79 ppm

**E $X_4$ A R A-$NH_2$ (18)** [SEQ ID NO.:4]

In this compound $X_4$ is the fluorinated amino acid of formula (Ib) wherein R is H and n is 2.

Retention time: 0.876 min; MW (monoisotopic) calcd for $C_{30}H_{44}F_6N_{10}O_7$: 770.33
MS (ES+): 771.19 $[M + H]^+$, 386.17 $[M + 2H]^{+2}/2$; MS (ES-): 769.28 $[M - H]^{-19}F$ NMR (50 mM Tris, 1 mM DTT, 0.001% Triton® X-100, pH = 7.5): 12.90 ppm

**E $X_5$ A R A-$NH_2$ (19)** [SEQ ID NO.:5]

In this compound $X_5$ is the fluorinated amino acid of formula (Ib) wherein R is H and n is 3.

Retention time: 0.909 min; MW (monoisotopic) calcd for $C_{31}H_{46}F_6N_{10}O_7$: 784.35

MS (ES+): 785.24 $[M + H]^+$, 393.17 $[M + 2H]^{+2}/2$; MS (ES-): 783.35 $[M - H]^{-}$ $^{19}F$ NMR (50 mM Tris, I mM DTT, 0.001% Triton® X-100, pH = 7.5): 12.83 ppm

**E $X_0$ A R A-$NH_2$ (20)** [SEQ ID NO.:6]

In this compound $X_0$ is the fluorinated amino acid of formula (Ib) wherein R is H and n is 4.

Retention time: 0.950 min; MW (monoisotopic) calcd for $C_{32}H_{48}F_6N_{10}O_7$: 798.36

MS (ES+): 799.26 $[M + H]^+$, 400.17 $[M + 2H]^{+2}/2$; MS (ES-): 797.33 $[M - H]^{-}$ $^{19}F$ NMR (50 mM Tris, 1 mM DTT, 0.001% Triton® X-100, pH = 7.5): 12.78 ppm.

## Example 13

### Procedure for the solid phase synthesis of a library of peptides

The synthesis of peptides of Table 2 was performed on solid phase starting from the commercially achieved Fmoc-Ile-Rink Amide MBHA resin, 4-(2',4'-dimethoxyphenyl-Fmoc-isoleucyl-aminomethyl)-phenoxyacetamido-methylbenzydrylamine copoly(styrene-1%DVB). The parallelization was obtained working on four 24 reactors Bohdan Miniblocks®.

The procedure for each elongation step is summarized as follows (3 ml/reactor):

1. Swelling of the resin with DCM for 30 minutes and DMA for 10 minutes
2. Fmoc deprotection with piperidine/DMA 20%, 3 times for 1, 5 and 20 minutes, respectively
3. Thoroughly washing with DMA x 4, DCM x 2, MeOH, DCM, MeOH, DCM, MeOH, DCM x 3, DMA x 2
4. Double coupling with 2 equivalents of the opportune Fmoc-AA-OH + 2 equiv. of PyBOP + 10 equiv. of DIEA + 3 ml of DMA, for 75 - 120 minutes each
5. Washing with DMA x 4
6. Capping with DMA/Acetic anhydride/DIEA 80:15:5, 2 times for 5 and 10 minutes respectively
7. Thoroughly washing with DMA x 4, DCM x 2, MeOH, DCM, MeOH, DCM, MeOH, DCM x 4

The last elongation step is for all sequences the coupling with the fluorinated amino acid 14 of Scheme 6.

After the final Fmoc deprotection, all peptides were cleaved from the resin as C-terminal amides with TFA/TIS/$H_2O$ 95/2.5/2.5, 3 ml for 90 minutes and 1 ml for 15 minutes. All cleavage solutions were collected in tared tubes together with two 10 minutes DCM washes with 1.5 ml and 2 ml, respectively.

The obtained peptide sequences and their identification LC/MS data are listed hereafter in Table 2:

Table 2

| SEQ ID NO | Sequence | Rt (min) | MW | Mono isotopic MW | $[M+2H]^{+2}/2$ | $[M + H]^+$ |
|---|---|---|---|---|---|---|
| [SEQ ID NO.:7] | $X_0$ S A V R I-$NH_2$ | 8.61 | 897.97 | 897.47 | 449.98 | 898.71 |
| [SEQ ID NO.:8] | $X_0$ E A V R I-$NH_2$ | 8.81 | 940.01 | 939.48 | 470.96 | 940.76 |
| [SEQ ID NO.:9] | $X_0$ S A K R I-$NH_2$ | 7.59 | 927.01 | 926.49 | 464.42 | 927.67 |
| [SEQ ID NO:10] | $X_0$ E A K R I-NH, | 7.63 | 969.05 | 968.50 | 485.46 | 969.84 |
| [SEQ ID NO:11] | $X_0$ S A G R I-$NH_2$ | 8.15 | 855.89 | 855.42 | 428.87 | 856.53 |
| [SEQ ID NO:12] | $X_0$ E A G R I-$NH_2$ | 8.29 | 897.93 | 897.43 | 449.85 | 898.64 |
| [SEQ ID NO:13] | $X_0$ S F V R I-$NH_2$ | 9.59 | 974.07 | 973.50 | 487.92 | 974.70 |
| [SEQ ID NO:14] | $X_0$ E F V R I-$NH=_2$ | 9.70 | 1016.11 | 1015.51 | 508.90 | 1016.94 |
| [SEQ ID NO:15] | $X_0$ S F K R I-$NH=_2$ | 8.42 | 1003.11 | 1002.52 | 502.43 | 1003.79 |
| [SEQ ID NO: 16] | $X_0$ E F K R I-$NH_2$ | 8.46 | 1045.15 | 1044.53 | 523.47 | 1045.83 |
| [SEQ ID NO:17] | $X_0$ S F G R I-$NH_2$ | 9.32 | 931.99 | 931.45 | 466.88 | 932.66 |
| [SEQ ID NO:18] | $X_0$ E F G R I-$NH_2$ | 9.41 | 974.03 | 973.46 | 487.86 | 974.77 |
| [SEQ ID NO: 19] | $X_0$ S L V R I-$NH_2$ | 9.45 | 940.05 | 939.51 | 470.89 | 940.69 |
| [SEQ ID NO:20] | $X_0$ E L V R I-$NH_2$ | 9.52 | 982.09 | 981.52 | 491.94 | 982.74 |
| [SEQ ID NO:21] | $X_0$ S L K R I-$NH_2$ | 8.25 | 969.09 | 968.54 | 485.40 | 969.71 |

(continued)

| SEQ ID NO | Sequence | Rt (min) | MW | Mono isotopic MW | $[M + 2H]^{+2}/2$ | $[M + H]^+$ |
|---|---|---|---|---|---|---|
| [SEQ ID NO:22] | $X_0$ E L K R I-$NH_2$ | 8.25 | 1011.13 | 1010.55 | 506.44 | 1011.82 |
| [SEQ ID NO:23] | $X_0$ S L G R I-$NH_2$ | 9.04 | 897.97 | 897.47 | 449.85 | 898.58 |
| [SEQ ID NO:24] | $X_0$ E L G R I-$NH_2$ | 9.15 | 940.01 | 939.48 | 470.89 | 940.69 |
| [SEQ ID NO:25] | $X_0$ S Q V R I-$NH_2$ | 8.48 | 955.02 | 954.49 | 478.34 | 955.66 |
| [SEQ ID NO:26] | $X_0$ E Q V R I-$NH_2$ | 8.68 | 997.06 | 996.50 | 499.38 | 997.90 |
| [SEQ ID NO:27] | $X_0$ S Q K R I-$NH_2$ | 7.40 | 984.07 | 983.51 | 492.84 | 984.74 |
| [SEQ ID NO:28] | $X_0$ E Q K R I-$NH_2$ | 7.53 | 1026.10 | 1025.52 | 513.89 | 1026.85 |
| [SEQ ID NO:29] | $X_0$ S Q G R I-$NH_2$ | 7.92 | 912.94 | 912.44 | 457.36 | 913.54 |
| [SEQ ID NO:30] | $X_0$ E Q G R I-$NH_2$ | 8.08 | 954.98 | 954.45 | 478.34 | 955.79 |
| [SEQ ID NO:31] | $X_0$ R V A E I-$NH_2$ | 8.91 | 940.01 | 939.48 | 471.10 | 940.88 |
| [SEQ ID NO:32] | $X_0$ R V A S I-$NH_2$ | 8.84 | 897.97 | 897.47 | 450.12 | 898.84 |
| [SEQ ID NO:33] | $X_0$ R K A E I-$NH_2$ | 8.13 | 969.05 | 968.50 | 485.61 | 969.90 |
| [SEQ ID NO:34] | $X_0$ R K A S I-$NH_2$ | 8.02 | 927.01 | 926.49 | 464.63 | 927.93 |
| [SEQ ID NO:35] | $X_0$ R G A E I-$NH_2$ | 8.59 | 897.93 | 897.43 | 450.12 | 898.71 |
| [SEQ ID NO:36] | $X_0$ R G A S I-$NH_2$ | 8.47 | 855.89 | 855.42 | 429.06 | 856.67 |
| [SEQ ID NO:37] | $X_0$ R V F E I-$NH_2$ | 10.12 | 1016.11 | 1015.51 | 509.13 | 1016.93 |
| [SEQ ID NO:38] | $X_0$ R V F S I-$NH_2$ | 10.07 | 974.07 | 973.50 | 488.01 | 974.89 |
| [SEQ ID NO:39] | $X_0$ R K F E I-$NH_2$ | 9.06 | 1045.15 | 1044.53 | 523.70 | 1045.95 |
| [SEQ ID NO:40] | $X_0$ R K F S I-$NH_2$ | 8.97 | 1003.11 | 1002.52 | 502.72 | 1003.91 |
| [SEQ ID NO:41] | $X_0$ R G F E I-$NH_2$ | 9.85 | 974.03 | 973.46 | 488.14 | 974.96 |
| [SEQ ID NO:42] | $X_0$ R G F S I-$NH_2$ | 9.69 | 931.99 | 931.45 | 467.09 | 932.85 |
| [SEQ ID NO:43] | $X_0$ R V L E I-$NH_2$ | 9.83 | 982.09 | 981.52 | 492.16 | 982.85 |
| [SEQ ID NO:44] | $X_0$ R V L S I-$NH_2$ | 9.72 | 940.05 | 939.51 | 471.17 | 940.88 |
| [SEQ ID NO:45] | $X_0$ R K L E I-$NH_2$ | 8.87 | 1011.13 | 1010.55 | 506.67 | 1012.01 |
| [SEQ ID NO:46] | $X_0$ R K L S I-$NH_2$ | 8.74 | 969.09 | 968.54 | 485.61 | 969.90 |
| [SEQ ID NO:47] | $X_0$ R G L E I-$NH_2$ | 9.69 | 940.01 | 939.48 | 471.10 | 940.82 |
| [SEQ ID NO:48] | $X_0$ R G L S I-$NH_2$ | 9.52 | 897.97 | 897.47 | 450.24 | 898.78 |
| [SEQ ID NO:49] | $X_0$ R V Q E I-$NH_2$ | 8.68 | 997.06 | 996.50 | 499.61 | 997.95 |
| [SEQ ID NO:50] | $X_0$ R V Q S I-$NH_2$ | 8.52 | 955.02 | 954.49 | 478.62 | 955.85 |
| [SEQ ID NO:51] | $X_0$ R K Q E I-$NH_2$ | 7.96 | 1026.10 | 1025.52 | 514.18 | 1026.97 |
| [SEQ ID NO:52] | $X_0$ R K Q S I-$NH_2$ | 7.85 | 984.07 | 969.50 | 493.19 | 984.80 |
| [SEQ ID NO:53] | $X_0$ R G Q E I-$NH_2$ | 8.31 | 954.98 | 954.45 | 478.62 | 955.91 |
| [SEQ ID NO:54] | $X_0$ R G Q S I-$NH_2$ | 8.16 | 912.94 | 912.44 | 457.56 | 913.81 |
| [SEQ ID NO:55] | $X_0$ A D R V S I-$NH_2$ | 8.52 | 1013.06 | 1012.49 | 507.33 | 1013.45 |
| [SEQ ID NO:56] | $X_0$ A E R V S I-$NH_2$ | 8.48 | 1027.09 | 1026.51 | 514.39 | 1027.43 |
| [SEQ ID NO:57] | $X_0$ A D R K S I-$NH_2$ | 7.67 | 1042.10 | 1041.52 | 521.90 | 1042.46 |
| [SEQ ID NO:58] | $X_0$ A E R K S I-$NH_2$ | 7.66 | 1056.13 | 1055.54 | 528.90 | 1056.44 |
| [SEQ ID NO:59] | $X_0$ A D R G S I-$NH_2$ | 8.03 | 970.98 | 970.45 | 486.35 | 971.35 |

(continued)

| SEQ ID NO | Sequence | Rt (min) | MW | Mono isotopic MW | [M + 2H]$^{+2}$/2 | [M + H]$^{+}$ |
|---|---|---|---|---|---|---|
| [SEQ ID NO:50] | $X_0$ A E R G S I-$NH_2$ | 8.03 | 985.01 | 984.46 | 493.34 | 985.41 |
| [SEQ ID NO:61] | $X_0$ F D R V S I-$NH_2$ | 9.16 | 1089.16 | 1088.52 | 545.41 | 1089.47 |
| [SEQ ID NO:62] | $X_0$ F E R V S I-$NH_2$ | 9.06 | 1103.19 | 1102.54 | 552.41 | 1103.45 |
| [SEQ ID NO:63] | $X_0$ F D R K S I-$NH_2$ | 8.30 | 1118.20 | 1117.55 | 559.92 | 1118.47 |
| [SEQ ID NO:64] | $X_0$ F E R K S I-$NH_2$ | 8.23 | 1132.23 | 1131.57 | 566.91 | 1132.53 |
| [SEQ ID NO:65] | $X_0$ F D R G S I-$NH_2$ | 8.72 | 1047.08 | 1046.48 | 524.37 | 1047.38 |
| [SEQ ID NO:66] | $X_0$ F E R G S I-$NH_2$ | 8.67 | 1061.11 | 1060.49 | 531.36 | 1061.43 |
| [SEQ ID NO:67] | $X_0$ L D R V S I-$NH_2$ | 8.97 | 1055.14 | 1054.54 | 528.38 | 1055.47 |
| [SEQ ID NO:68] | $X_0$ L E R V S I-$NH_2$ | 8.92 | 1069.17 | 1068.56 | 535.44 | 1069.46 |
| [SEQ ID NO:69] | $X_0$ L D R K S I-$NH_2$ | 8.02 | 1084.18 | 1083.57 | 542.89 | 1084.48 |
| [SEQ ID NO:70] | $X_0$ L E R K S I-$NH_2$ | 8.01 | 1098.21 | 1097.58 | 549.95 | 1098.47 |
| [SEQ ID NO:71] | $X_0$ L D R G S I-$NH_2$ | 8.46 | 1013.06 | 1012.49 | 507.33 | 1013.45 |
| [SEQ ID NO:72] | $X_0$ L E R G S I-$NH_2$ | 8.44 | 1027.09 | 1026.51 | 514.39 | 1027.43 |
| [SEQ ID NO:73] | $X_0$ Q D R V S I-$NH_2$ | 8.43 | 1070.11 | 1069.51 | 535.83 | 1070.49 |
| [SEQ ID NO:74] | $X_0$ Q E R V S I-$NH_2$ | 8.38 | 1084.14 | 1083.53 | 542.89 | 1084.42 |
| [SEQ ID NO:75] | $X_0$ Q D R K S I-$NH_2$ | 7.60 | 1099.16 | 1098.54 | 550.40 | 1099.44 |
| [SEQ ID NO:76] | $X_0$ Q E R K S I-$NH_2$ | 7.61 | 1113.18 | 1112.56 | 557.39 | 1113.42 |
| [SEQ ID NO:77] | $X_0$ Q D R G S I-$NH_2$ | 7.94 | 1028.03 | 1027.47 | 514.85 | 1028.40 |
| [SEQ ID NO:78] | $X_0$ Q E R G S I-$NH_2$ | 7.94 | 1042.06 | 1041.48 | 521.84 | 1042.46 |
| [SEQ ID NO:79] | $X_0$ V R D A S I-$NH_2$ | 8.22 | 1013.06 | 1012.49 | 507.33 | 1013.45 |
| [SEQ ID NO:80] | $X_0$ V R E A S I-$NH_2$ | 8.28 | 1027.09 | 1026.51 | 514.39 | 1027.43 |
| [SEQ ID NO: 81] | $X_0$ K R D A S I-$NH_2$ | 7.86 | 1042.10 | 1041.52 | 521.90 | 1042.46 |
| [SEQ ID NO:82] | $X_0$ K R E A S I-$NH_2$ | 7.84 | 1056.13 | 1055.54 | 528.90 | 1056.51 |
| [SEQ ID NO:83] | $X_0$ G R D A S I-$NH_2$ | 8.15 | 970.98 | 970.45 | 486.35 | 971.42 |
| [SEQ ID NO:84] | $X_0$ G R E A S I-$NH_2$ | 8.18 | 985.01 | 984.46 | 493.34 | 985.41 |
| [SEQ ID NO:85] | $X_0$ V R D F S I-$NH_2$ | 9.18 | 1089.16 | 1088.52 | 545.41 | 1089.47 |
| [SEQ ID NO:86] | $X_0$ V R E F S I-$NH_2$ | 9.32 | 1103.19 | 1102.54 | 552.41 | 1103.45 |
| [SEQ ID NO:87] | $X_0$ K R D F S I-$NH_2$ | 8.66 | 1118.20 | 1117.55 | 559.92 | 1118.47 |
| [SEQ ID NO:88] | $X_0$ K R E F S I-$NH_2$ | 8.79 | 1132.23 | 1131.57 | 566.91 | 1132.46 |
| [SEQ ID NO:89] | $X_0$ G R D F S I-$NH_2$ | 9.01 | 1047.08 | 1046.48 | 524.37 | 1047.44 |
| [SEQ ID NO:90] | $X_0$ G R E F S I-$NH_2$ | 9.12 | 1061.11 | 1060.49 | 531.36 | 1061.43 |
| [SEQ ID NO:91] | $X_0$ V R D L S I-$NH_2$ | 8.94 | 1055.14 | 1054.54 | 528.38 | 1055.47 |
| [SEQ ID NO:92] | $X_0$ V R E L S I-$NH_2$ | 9.08 | 1069.17 | 1068.56 | 535.44 | 1069.46 |
| [SEQ ID NO:93] | $X_0$ K R D L S I-$NH_2$ | 8.41 | 1084.18 | 1083.57 | 542.95 | 1084.48 |
| [SEQ ID NO:94] | $X_0$ K R E L S I-$NH_2$ | 8.55 | 1098.21 | 1097.58 | 549.95 | 1098.53 |
| [SEQ ID NO:95] | $X_0$ G R D L S I-$NH_2$ | 8.73 | 1013.06 | 1012.49 | 507.33 | 1013.38 |
| [SEQ ID NO:96] | $X_0$ G R E L S I-$NH_2$ | 8.87 | 1027.09 | 1026.51 | 514.39 | 1027.37 |
| [SEQ ID NO:971 | $X_0$ V R D Q S I-$NH_2$ | 8.05 | 1070.11 | 1069.51 | 535.89 | 1070.49 |

(continued)

| SEQ ID NO | Sequence | Rt (min) | MW | Mono isotopic MW | $[M + 2H]^{+2}/2$ | $[M + H]^+$ |
|---|---|---|---|---|---|---|
| [SEQ ID NO:98] | $X_0$ V R E Q S I-$NH_2$ | 8.06 | 1084.14 | 1083.53 | 542.89 | 1084.48 |
| [SEQ ID NO:99] | $X_0$ K R D Q S I-$NH_2$ | 7.75 | 1099.16 | 1098.54 | 550.40 | 1099.50 |
| [SEQ ID NO:100] | $X_0$ K R E Q S I-$NH_2$ | 7.72 | 1113.18 | 1112.56 | 557.39 | 1113.49 |
| [SEQ ID NO:101] | $X_0$ G R D Q S I-$NH_2$ | 8.06 | 1028.03 | 1027.47 | 514.85 | 1028.40 |
| [SEQ ID NO: 102] | $X_0$ G R E Q S I-$NH_2$ | 8.07 | 1042.06 | 1041.48 | 521.84 | 1042.39 |

wherein $X_0$ is the fluorinated amino acid of the following formula (Ic):

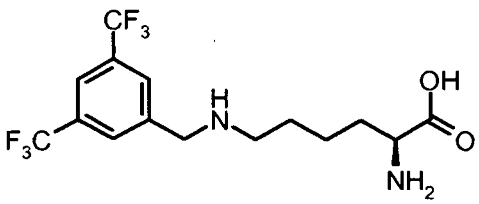

**(Ic)**

## Claims

**1.** A compound of the formula (1):

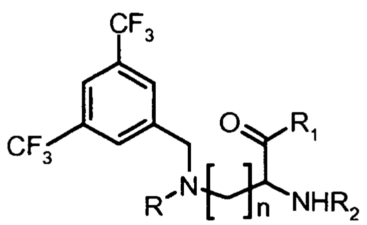

**(I)**

wherein:

R is hydrogen atom, a N-protecting group or 3,5-bis(trifluoromethyl)benzyl group;
$R_1$ is hydroxy, $C_1$-$C_4$ alkyl group, $C_1$-$C_4$ alkoxy or aryl $C_1$-$C_4$ alkyloxy group, or the N terminus of any $\alpha$-amino acid or peptide sequence;
$R_2$ is hydrogen atom, a N-protecting group or the C terminus of any $\alpha$-amino acid or peptide sequence; n is 1, 2, 3 or 4, or a salt thereof.

**2.** A compound of the formula (I) according to claim 1, **characterized in that** R is hydrogen atom, 3,5-bis(trifluoromethyl) benzyl group, t-butoxycarbonyl group, 9-fluorenylmethoxycarbonyl group; $R_1$ is hydroxy or the N terminus of any $\alpha$-amino acid or peptide sequence; $R_2$ is hydrogen atom, t-butoxycarbonyl group, 9-fluorenylmethoxycarbonyl group or the C terminus of any $\alpha$-amino acid or peptide sequence and n is 2, 3 or 4.

**3.** A compound of the formula (I) according to claim 1, **characterized in that** R is hydrogen atom, t-butoxycarbonyl or 9-fluorenylmethoxycarbonyl group.

**4.** A compound of the formula (I) according to claim 1, **characterized in that** $R_1$ is the N terminus of any α-amino acid or peptide sequence, or a salt thereof.

**5.** A compound of the formula (I) according to claim 1, **characterized in that** $R_2$ is the C terminus of any α-amino acid or peptide sequence, or a salt thereof.

**6.** A compound of the formula (I) according to claim 1, **characterized in that** $R_1$ is the N terminus of any α-amino acid or peptide sequence, and $R_2$ is the C terminus of any α-amino acid or peptide sequence or hydrogen atom, or a salt thereof.

**7.** A compound of the formula (I) according to claim 6, **characterized in that** $R_1$ is the N terminus of any α-amino acid or peptide sequence, and $R_2$ is the C terminus of any α-amino acid or peptide sequence, or a salt thereof.

**8.** A compound of the formula (I) according to claim 6, **characterized in that** $R_1$ is the N terminus of a peptide sequence, and $R_2$ is the C terminus of any α-amino acid or hydrogen atom.

**9.** A process for preparing a compound of formual (I) as defined in claim 1, which process comprises:

a) converting a compound of the formula (II):

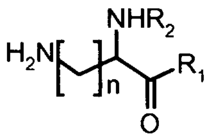

**(II)**

wherein $R_1$ is hydroxy, $R_2$ is a N-protecting group and n is 1, 2, 3 or 4 into a corresponding compound of the formula (I) wherein R is as defined in claim 1, $R_1$ is hydroxy, $R_2$ is a N-protecting group and n is 1, 2, 3 or 4, through reductive amination with the properly choosen aldehyde and a reducing agent or alkylation with the corresponding benzyl halide in the presence of a base and either
b) installing a suitable orthogonal protective group on a resultant compound of the formula (I) when R is hydrogen atom so as to obtain a compound of the formula (I) wherein R is a N-protecting group; or
c) salifying a resultant compound of the formula (I) when R is 3,5-bis(trifluoromethyl)benzyl group with the appropriate acidic counterpart and
d) condensing the resultant compounds of the formula (I) with suitable protected amino acids, aminoacid derivatives or peptides, in the order of the amino acids of the desired final compound of the formula (I), either by liquid phase methodology or solid phase synthesis, operating by standard strategies and conditions, so as to obtain another compound of the formula (I) wherein either $R_1$ is the N terminus of any α-amino acid or peptide sequence, or $R_2$ is the C terminus of any α-amino acid or peptide sequence, and optionally converting the resulting compound of the formula (I) into a salt thereof.

**10.** A method of using a compound of formula (I) as defined in claim 1 for detecting through "Fluorine chemical shift Anisotropy and eXchange for Screening" (FAXS) experiments weak binders, for identifying inhibitors of an enzymatic reaction or the most efficient substrate for an enzyme by means of "three Fluorine Atoms for Biochemical Screening" (3-FABS) method.

**Patentansprüche**

**1.** Verbindung der Formel (I)

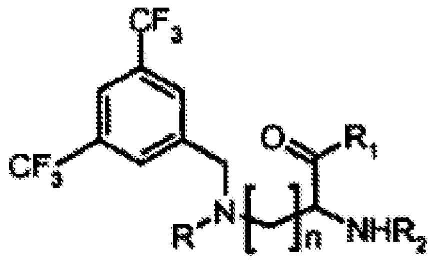

(I)

wobei:

R für ein Wasserstoffatom, eine N-Schutzgruppe oder eine 3,5-Bis(trifluormethyl)benzyl-Gruppe steht;
$R_1$ für eine Hydroxygruppe, eine $C_1$-$C_4$-Alkylgruppe, eine $C_1$-$C_4$-Alkoxy- oder Aryl-$C_1$-$C_4$-alkyloxygruppe oder das N-Ende irgendeiner α-Aminosäure oder Peptidsequenz steht;
$R_2$ für ein Wasserstoffatom, eine N-Schutzgruppe oder das C-Ende irgendeiner α-Aminosäure oder Peptidsequenz steht und n für 1, 2, 3 oder 4 steht; oder ein Salz derselben.

2. Verbindung der Formel (I) nach Anspruch 1, **dadurch gekennzeichnet, dass** R für ein Wasserstoffatom, eine 3,5-Bis(trifluormethyl)benzyl-Gruppe, eine t-Butoxycarbonyl-Gruppe oder eine 9-Fluorenylmethoxycarbonyl-Gruppe steht; $R_1$ für eine Hydroxygruppe oder das N-Ende irgendeiner α-Aminosäure oder Peptidsequenz steht; $R_2$ für ein Wasserstoffatom, eine t-Butoxycarbonyl-Gruppe, eine 9-Fluorenylmethoxycarbonyl-Gruppe oder das C-Ende irgendeiner α-Aminosäure oder Peptidsequenz steht und n für 2, 3 oder 4 steht.

3. Verbindung der Formel (I) nach Anspruch 1, **dadurch gekennzeichnet, dass** R für ein Wasserstoffatom, eine t-Butoxycarbonyl-Gruppe oder eine 9-Fluorenylmethoxycarbonyl-Gruppe steht.

4. Verbindung der Formel (I) nach Anspruch 1, **dadurch gekennzeichnet, dass** $R_1$ für das N-Ende irgendeiner α-Aminosäure oder Peptidsequenz steht, oder ein Salz derselben.

5. Verbindung der Formel (I) nach Anspruch 1, **dadurch gekennzeichnet, dass** $R_2$ für das C-Ende irgendeiner α-Aminosäure oder Peptidsequenz steht, oder ein Salz derselben.

6. Verbindung der Formel (I) nach Anspruch 1, **dadurch gekennzeichnet, dass** $R_1$ für das N-Ende irgendeiner α-Aminosäure oder Peptidsequenz steht und $R_2$ für das C-Ende irgendeiner α-Aminosäure oder Peptidsequenz oder für ein Wasserstoffatom steht, oder ein Salz derselben.

7. Verbindung der Formel (I) nach Anspruch 6, **dadurch gekennzeichnet, dass** $R_1$ für das N-Ende irgendeiner α-Aminosäure oder Peptidsequenz steht und $R_2$ für das C-Ende irgendeiner α-Aminosäure oder Peptidsequenz steht, oder ein Salz derselben.

8. Verbindung der Formel (I) nach Anspruch 6, **dadurch gekennzeichnet, dass** $R_1$ für das N-Ende irgendeiner α-Aminosäure oder Peptidsequenz steht und $R_2$ für das C-Ende irgendeiner α-Aminosäure oder Peptidsequenz steht.

9. Verfahren zur Herstellung einer Verbindung der Formel (I) gemäß der Definition in Anspruch 1, wobei das Verfahren das Folgende umfasst:

a) Umwandeln einer Verbindung der Formel (II)

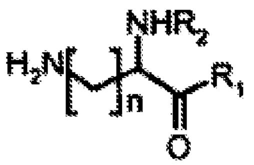

(II)

wobei $R_1$ für eine Hydroxygruppe steht, $R_2$ für eine N-Schutzgruppe steht und n für 1, 2, 3 oder 4 steht, in eine entsprechende Verbindung der Formel (I), wobei R wie in Anspruch 1 definiert ist, $R_1$ für eine Hydroxygruppe steht, $R_2$ für eine N-Schutzgruppe steht und n für 1, 2, 3 oder 4 steht, durch reduktive Aminierung mit dem entsprechend ausgewählten Aldehyd und einem Reduktionsmittel oder Alkylierung mit dem entsprechenden Benzylhalogenid in Gegenwart einer Base und entweder

b) Anbringen einer geeigneten orthogonalen Schutzgruppe an einer resultierenden Verbindung der Formel (I), wenn R für ein Wasserstoffatom steht, um eine Verbindung der Formel (I) zu erhalten, wobei R für eine N-Schutzgruppe steht; oder

c) Überführen einer resultierenden Verbindung der Formel (I) in ein Salz mit dem entsprechenden sauren Gegenstück, wenn R für eine 3,5-Bis(trifluormethyl)benzyl-Gruppe steht, und

d) Kondensieren der resultierenden Verbindungen der Formel (I) mit geeigneten geschützten Aminosäuren, Aminosäurederivaten oder Peptiden in der Reihenfolge der Aminosäuren der gewünschten Endverbindung der Formel (I), entweder über die Flüssigphasenmethodik oder über eine Festphasensynthese, wobei mit Standardstrategien und -bedingungen gearbeitet wird, um eine andere Verbindung der Formel (I) zu erhalten, wobei entweder $R_1$ für das N-Ende irgendeiner $\alpha$-Aminosäure oder Peptidsequenz steht oder $R_2$ für das C-Ende irgendeiner $\alpha$-Aminosäure oder Peptidsequenz steht, und gegebenenfalls Umwandeln der resultierenden Verbindung der Formel (I) in ein Salz derselben.

**10.** Verfahren zur Verwendung einer Verbindung der Formel (I) gemäß der Definition in Anspruch 1 zum Nachweis von schwachen Bindern über FAXS-Experimente ("Fluorine chemical shift Anisotropy and eXchange for Screening"), um Inhibitoren einer enzymatischen Reaktion oder das effektivste Substrat für ein Enzym mittels des 3-FABS-Verfahrens ("3 Fluorine Atoms for Biochemical Screening") zu identifizieren.

## Revendications

**1.** Composé de formule (I) :

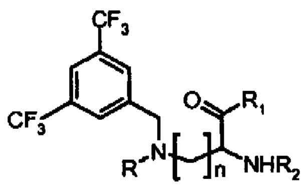

(I)

où

R est un atome d'hydrogène, un groupe N-protecteur ou un groupe 3,5-bis(trifluorométhyl)benzyle ;
$R_1$ est un hydroxy, un groupe alkyle en $C_1$-$C_4$, un groupe alcoxy en $C_1$-$C_4$ ou un groupe arylalkyloxy en $C_1$-$C_4$, ou le N-terminal d'une quelconque séquence d'acides $\alpha$-aminés ou peptidique ;
$R_2$ est un atome d'hydrogène, un groupe N-protecteur ou le C-terminal d'une quelconque séquence d'acides $\alpha$-aminés ou peptidique ; n vaut 1, 2, 3 ou 4, ou sel de celui-ci.

**2.** Composé de formule (I) selon la revendication 1, **caractérisé en ce que** R est un atome d'hydrogène, un groupe 3,5-bis(trifluorométhyl)benzyle, un groupe t-butoxycarbonyle, un groupe 9-fluorénylméthoxycarbonyle ; $R_1$ est un hydroxy ou le N-terminal d'une quelconque séquence d'acides $\alpha$-aminés ou peptidique ; $R_2$ est un atome d'hydrogène, un groupe t-butoxycarbonyle, un groupe 9-fluorénylméthoxycarbonyle ou le C-terminal d'une quelconque séquence d'acides $\alpha$-aminés ou peptidique et n vaut 2, 3 ou 4.

**3.** Composé de formule (I) selon la revendication 1, **caractérisé en ce que** R est un atome d'hydrogène, un groupe t-butoxycarbonyle ou un groupe 9-fluorénylméthoxycarbonyle.

**4.** Composé de formule (I) selon la revendication 1, **caractérisé en ce que** $R_1$ est le N-terminal d'une quelconque séquence d'acides $\alpha$-aminés ou peptidique, ou sel de celui-ci.

**5.** Composé de formule (I) selon la revendication 1, **caractérisé en ce que** $R_2$ est le C-terminal d'une quelconque

séquence d'acides α-aminés ou peptidique, ou sel de celui-ci.

**6.** Composé de formule (I) selon la revendication 1, **caractérisé en ce que** $R_1$ est le N-terminal d'une quelconque séquence d'acides α-aminés ou peptidique, et $R_2$ est le C-terminal d'une quelconque séquence d'acides α-aminés ou peptidique ou un atome d'hydrogène, ou sel de celui-ci.

**7.** Composé de formule (I) selon la revendication 6, **caractérisé en ce que** $R_1$ est le N-terminal d'une quelconque séquence d'acides α-aminés ou peptidique, et $R_2$ est le C-terminal d'une quelconque séquence d'acides α-aminés ou peptidique, ou sel de celui-ci.

**8.** Composé de formule (I) selon la revendication 6, **caractérisé en ce que** $R_1$ est le N-terminal d'une séquence peptidique, et $R_2$ est le C-terminal d'une quelconque séquence d'acides α-aminés ou un atome d'hydrogène.

**9.** Procédé de préparation d'un composé de formule (I) tel que défini dans la revendication 1, lequel procédé comprend :

a) la conversion d'un composé de formule (II) :

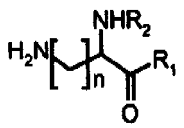

(II)

où $R_1$ est un hydroxy, $R_2$ est un groupe N-protecteur et n vaut 1, 2, 3 ou 4, en un composé correspondant de formule (I) où R est tel que défini dans la revendication 1, $R_1$ est un hydroxy, $R_2$ est un groupe N-protecteur et n vaut 1, 2, 3 ou 4, par amination réductrice avec l'aldéhyde adéquatement choisi et un agent réducteur ou par alkylation avec l'halogénure de benzyle correspondant en présence d'une base et soit
b) l'installation d'un groupe protecteur orthogonal approprié sur un composé obtenu de formule (I) lorsque R est un atome d'hydrogène de façon à obtenir un composé de formule (I) où R est un groupe N-protecteur ; soit
c) la salification d'un composé obtenu de formule (I) lorsque R est un groupe 3,5-bis(trifluorométhyl)benzyle avec l'homologue acide approprié et
d) la condensation des composés obtenus de formule (I) avec les acides aminés protégés appropriés, les dérivés aminoacides ou les peptides, dans l'ordre des acides aminés du composé final souhaité de formule (I), soit par le biais d'une méthode en phase liquide soit par synthèse en phase solide, en utilisant des stratégies et des conditions normalisées, de façon à obtenir un autre composé de formule (I) où soit $R_1$ est le N-terminal d'une quelconque séquence d'acides α-aminés ou peptidique, soit $R_2$ est le C-terminal d'une quelconque séquence d'acides α-aminés ou peptidique, et facultativement la conversion du composé obtenu de formule (I) en un sel de celui-ci.

**10.** Procédé d'utilisation d'un composé de formule (I) tel que défini dans la revendication 1, pour détecter, par le biais d'expériences FAXS (anisotropie de déplacement chimique du fluor et échange pour le criblage), les liants faibles, pour identifier les inhibiteurs d'une réaction enzymatique ou le substrat le plus efficace pour une enzyme au moyen de la méthode 3-FABS (trois atomes de fluors pour le criblage biochimique).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004051214 A
- WO 20050005978 A
- WO 2003045378 A

### Non-patent literature cited in the description


- **Smits, R et al.** *Current Topics in Medicinal Chemistry,* July 2006, vol. 6 (14), 1483-1498
- **Dalvit, Claudio et al.** Rapid NMR-based functional screening and IC50 measurements performed at unprecedentedly low enzyme concentration. *Drug Development Research,* 2005, vol. 64, 105-113
- **Bodanszky et al.** Peptide Synthesis. Interscience Publishers, 1966
- Protective Group in Organic Chemistry. Plenum Press, 1973
- **Barany et al.** The Peptides: Analysis, Synthesis, Biology. Academic Press, 1980
- **J. Stewart ; J. Young.** Solid phase peptide synthesis. Pierce Chemical Company, 1984
- **E. Atherthon ; R. C. Sheppard.** Solid phase peptide synthesis: a practical approach. IRL Press, 1989
- Solid-phase synthesis. A practical guide. Marcel Decker, 2000
- Fmoc solid phase peptide synthesis: a practical approach. Oxford University Press, 2000
- **de Macédo, P. ; Marrano, C. ; Keillor, J. W.** *Bioorg. Med. Chem.,* 2002, vol. 10, 355-360
- **Sakura, N. ; Itoh, T. ; Uchida, Y. ; Ohki, K. ; Okimura, K. ; Chiba, K. ; Sato, Y. ; Sawanishi, H.** *Bull. Chem. Soc. Japan,* 2004, vol. 77, 1915-1924
- **Varghese, J.** *PCT Int. Appl.,* 2003, 204
- **Banwell, M. G. ; McRae, K. J.** *J. Oil. Chem.,* 2001, vol. 66, 6768-6774
- **Boyle, W. J., Jr ; Sifniades, S. ; Van Peppen, J. F.** *J. Org. Chem.,* 1979, vol. 44, 4841-4847
- *Eur. J. Biochem.,* 1984, vol. 138, 9-37